(19) Europäisches Patentamt
European Patent Office
Office européen des brevets

(11) **EP 0 793 541 B1**

(12) **EUROPÄISCHE PATENTSCHRIFT**

(45) Veröffentlichungstag und Bekanntmachung des Hinweises auf die Patenterteilung:
**22.09.1999 Patentblatt 1999/38**

(21) Anmeldenummer: **95940937.6**

(22) Anmeldetag: **27.12.1995**

(51) Int. Cl.$^6$: **B05D 3/06**, C07C 271/20, C08F 2/50, G03F 7/031, C08F 290/04

(86) Internationale Anmeldenummer:
**PCT/CH95/00310**

(87) Internationale Veröffentlichungsnummer:
**WO 96/20795 (11.07.1996 Gazette 1996/31)**

(54) **NCO-TERMINALE VINYLTELOMERE**
NCO-TERMINATED VINYL TELOMERS
TELOMERES VINYLIQUES A GROUPES TERMINAUX NCO

(84) Benannte Vertragsstaaten:
**AT BE CH DE DK ES FR GB GR IE IT LI LU NL PT SE**

(30) Priorität: **30.12.1994 CH 396794**
**30.12.1994 CH 396894**

(43) Veröffentlichungstag der Anmeldung:
**10.09.1997 Patentblatt 1997/37**

(73) Patentinhaber:
- **Novartis AG**
**4058 Basel (CH)**
Benannte Vertragsstaaten:
**BE CH DE DK ES FR GB GR IE IT LI LU NL PT SE**
- **Novartis-Erfindungen Verwaltungsgesellschaft m.b.H.**
**1235 Wien (AT)**
Benannte Vertragsstaaten:
**AT**

(72) Erfinder:
- **CHABRECEK, Peter**
**Clayton, VIC 3169 (AU)**
- **LOHMANN, Dieter**
**4142 Münchenstein (CH)**
- **DIETLIKER, Kurt**
**1700 Fribourg (CH)**

(56) Entgegenhaltungen:
**EP-A- 0 281 941**
**EP-A- 0 302 831**
**EP-A- 0 632 329**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 793 541 B1

Printed by Xerox (UK) Business Services
2.16.7/3.6

## Beschreibung

Die Erfindung betrifft neue NCO-terminale Vinyltelomere, die sich insbesondere für die Modifizierung von Oberflächen und als Beschichtungsmaterial eignen, darüber hinaus aber auch zur Herstellung von polymerisierbaren Verbindungen oder segmentierten Copolymeren, die sich zu Polymeren umsetzen respektive in Formkörper überführen lassen, ferner Formkörper enthaltend derartige Polymere, ferner die Verwendung der Polymere zur Herstellung von Formkörpern und Verfahren zur Herstellung der Polymere und der Formkörper. Bevorzugte Formkörper sind ophthalmische Linsen, insbesondere Kontaktlinsen. Die Vinyltelomere zeichnen sich unter anderem dadurch gegenüber bekannten Vinyltelomeren aus, dass sie eine engere Molekulargewichtsverteilung der jeweiligen, aus dem oder den verwendeten Vinylmonomeren, gebildeten Kettenlängen aufweisen und dadurch, dass sie mit der besonders reaktiven Isocyanatgruppe mono-funktionalisiert sind.

Die EP-A-0 281 941 offenbart u.a. mit einer Isocyanatgruppe mono-funktionalisierte Photoinitiatoren, nicht jedoch deren Verwendung zur Herstellung von OCN-terminalen Vinyltelomeren.

Bei den erfindungsgemässen OCN-terminalen Vinyltelomeren handelt es sich um Verbindungen der Formel I,

$$O{=}C{=}N\text{-}PI^{*}\text{-}(A)_p\text{-}R_a \qquad (I)$$

worin

PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
$R_a$ für eine einwertige Gruppe steht, die geeignet ist, als Kettenabbrecher einer Polymerisation zu dienen, und p für eine ganze Zahl von 3 bis 500 steht.

Die Gruppe $-(A)_p-$ in Formel I bzw. die für deren Herstellung verwendeten Vinylmonomere enthalten vorzugsweise keine H-aktiven Gruppen.

Die erfindungsgemässen Vinyltelomere der Formel I lassen sich dadurch aufbauen, dass ein Photoinitiator der Formel B,

$$OCN\text{-}PI^{*}\text{-}R_{aa} \qquad (B)$$

worin PI* wie vorstehend definiert ist und $R_{aa}$ für den Teil eines Photoinititors steht, der bei einer Aufspaltung des Photoinitiators das weniger reaktive Radikal bildet, mit einem Vinylmonomer auf an sich bekannte Weise umgesetzt wird, das als Bestandteil "A" in das Vinyltelomere eingebaut wird, worin A wie vorstehend definiert ist. Der Kettenabbruch erfolgt beispielsweise durch das weniger reaktive Radikal des Photoinitiators $R_{aa}$ der Formel B oder durch andere geeignete Kettenabbrecher, die unter den Reaktionsbedingungen im Reaktionsgemisch vorliegen, wie z.B. H-Radikale oder OH-Radikale oder aus Lösungsmittel gebildete Radikale. Die Variable $R_a$ ist bevorzugt der Bestandteil $R_{aa}$ des Photoinitiators der Formel B.

Bei den erfindungsgemässen polymerisierbaren Verbindungen handelt es sich um Verbindungen der Formel C,

$$\text{Mono}\text{—}R_x\text{-}\overset{\overset{\large O}{\|}}{C}\text{-NH-}PI^{*}\text{-}(A)_p\text{-}R_a \qquad (C)$$

worin Mono für einen einwertigen Rest eines Vinylmonomeren steht, von dem die Gruppe $R_x$-H entfernt ist,

$R_x$ unabhängig voneinander für eine Bindung, -O-, $-NR_N-$ oder -S- steht, worin $R_N$ für Wasserstoff oder $C_1$-$C_6$-Alkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
$R_a$ für eine einwertige Gruppe steht, die geeignet ist, als Kettenabbrecher einer Polymerisation zu dienen, und p für eine ganze Zahl von 3 bis 500 steht.

Die Bedeutung "Bindung" für $R_x$ ist nur für den Fall relevant, dass eine Gruppe OH im einwertigen Rest eines Vinylmonomeren als Bestandteil einer COOH-Gruppe vorliegt. Eine COOH-Gruppe reagiert mit einer Isocyanat-Gruppe unter Abspaltung von $CO_2$ und unter Ausbildung einer Bindung "-CO-NH-". Nur in diesem Fall bedeutet $R_x$ eine Bindung im Reaktionsprodukt, nicht jedoch in einem Ausgangsprodukt enthaltend die Gruppe "$R_x$-H".

Die erfindungsgemässen polymerisierbaren Verbindungen der Formel C lassen sich dadurch aufbauen, dass ein Vinyltelomer der Formel I, wie vorstehend definiert, auf an sich bekannte Weise mit einem Vinylmonomer umgesetzt wird, das mindestens eine Gruppe $R_x$-H enthält, worin $R_x$ wie vorstehend definiert ist aber von einer Bindung verschieden ist.

Bei den erfindungsgemässen segmentierten Copolymeren handelt es sich um unvernetzte aber gegebenfalls vernetzbare Copolymere der Formel D,

$$\text{Macro}\left[R_x\text{-}\overset{\overset{\large O}{\|}}{C}\text{-NH-PI}^{*}\text{-}(A)_p\text{-}R_a\right]_m \qquad (D)$$

worin Macro für einen m-wertigen Rest eines Makromeren steht, von dem die Anzahl von m Gruppen $R_x$-H entfernt ist,

$R_x$ unabhängig voneinander für eine Bindung, -O-, -$NR_N$- oder -S- steht, worin $R_N$ für Wasserstoff oder $C_1$-$C_6$-Alkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
jedes $R_a$ unabhängig voneinander für eine einwertige Gruppe steht, die geeignet ist, als Kettenabbrecher einer Polymerisation zu dienen,
p unabhängig von m für eine ganze Zahl von 3 bis 500 steht und
m für eine ganze Zahl von 1 bis 100 steht.

Die Bedeutung "Bindung" für $R_x$ ist nur für den Fall relevant, dass eine Gruppe OH im Makromer als Bestandteil einer COOH-Gruppe vorliegt. Eine COOH-Gruppe reagiert mit einer Isocyanat-Gruppe unter Abspaltung von $CO_2$ und unter Ausbildung einer Bindung "-CO-NH-". Nur in diesem Fall bedeutet $R_x$ eine Bindung im Reaktionsprodukt, nicht jedoch in einem Ausgangsprodukt enthaltend die Gruppe "$R_x$-H".

Unter einem segmentierten Copolymer werden erfindungsgemäss Blockcopolymere` Pfropfcopolymere, insbesondere Kammcopolymere, oder Sterncopolymere verstanden.

Die erfindungsgemässen segmentierten Copolymere der Formel D lassen sich dadurch aufbauen, dass ein Makromer der Formel A:

$$\text{Macro-}(R_xH)_m \qquad (A)$$

worin Macro, $R_x$ und m wie vorstehend definiert sind aber $R_x$ von einer Bindung verschieden ist, mit einem Vinyltelomeren der Formel I auf an sich bekannte Weise umgesetzt wird,

$$O{=}C{=}N\text{-PI}^{*}\text{-}(A)_pR_a \qquad (I)$$

worin PI*, A, $R_a$ und p wie vorstehend definiert sind.

Das erfindungsgemäss geeignete Makromer der Formel A weist eine Anzahl von m Gruppen -$R_x$H auf, bei denen es sich um Hydroxygruppen (auch um solche, die Bestandteil einer Carboxylgruppe -COOH sind), Aminogruppen oder Niederalkylaminogruppen (auch um solche, die Bestandteil einer Amidgruppe -$CONR_N$ sind) oder Mercaptogruppen handelt. Diese Gruppen sind mit der Isocyanatgruppe des Photoinitiators der Formel B coreaktiv. Analoges gilt für die Vinylmonomere, die den Rest "Mono" in einer Verbindung der Formel C beisteuern.

Der Index p steht vorzugsweise für eine Zahl von 5 bis 200, insbesondere für eine Zahl von 10 bis 100.

Der Index m steht vorzugsweise für eine Zahl von 2 bis 15, insbesondere für eine Zahl von 2 bis 5.

Bei den erfindungsgemässen Polymeren handelt es sich um die Polymerisationsprodukte eines polymerisationsfähigen Gemisches, das die folgenden Bestandteile enthalt:

a) eine polymerisationsfähige Verbindung der Formel C wie vorstehend definiert,

b) gegebenenfalls ein copolymerisierbares Vinylmonomer

c) einen copolymerisierbaren Vernetzer; oder um die Polymerisationsprodukte eines polymerisationsfähigen Gemisches, das die folgenden Bestandteile enthält:

aa) eine polymerisationsfähige Verbindung der Formel D wie vorstehend definiert, worin im Bestandteil $-(A)_p-$ eine reaktive Gruppe enthalten ist,

bb) gegebenenfalls ein copolymerisierbares Vinylmonomer

cc) einen Vernetzer, der mit der reaktiven Gruppe im Bestandteil $-(A)_p-$ der Verbindung der Formel D coreaktiv ist.

In den genannten polymerisationsfähigen Gemischen wird eine Verbindung der Formel C oder D, falls ein copolymerisierbares Vinylmonomer verwendet wird, vorzugsweise in einer Menge von 10 bis 90 Gewichtsprozent eingesetzt, insbesondere von 20 bis 80 Gewichtsprozent, ein copolymerisierbares Vinylmonomer wird dann, d.h. falls anwesend, vorzugsweise in einer Menge von 10 bis 90 Gewichtsprozent, insbesondere von 20 bis 80 Gewichtsprozent, eingesetzt, wobei sich diese Gewichtsprozentangaben auf die Mengen der Komponenten a) und b) bzw. aa) und bb) relativ zueinander beziehen. Ein Vernetzer c) oder cc) wird vorzugsweise in einer Menge von bis zu 25 Gewichtsprozent, insbesondere in einer Menge von bis zu 12,5 Gewichtsprozent, eingesetzt, bezogen auf die Summe der Komponenten a) und b) beziehungsweise aa) und bb).

Bei einem copolymerisierbaren Vernetzer c), wie im vorstehenden Absatz erwähnt, handelt es sich um einen typischen oligovinylischen Vernetzer, wie im Stand der Technik bekannt. Bei einem Vernetzer cc), wie im vorstehenden Absatz erwähnt, handelt es sich um eine oligofunktionelle Verbindung, die mit im $-(A)_p-$ Teil enthaltenen reaktiven Gruppen coreaktiv ist.

Unter einer reaktiven Gruppe im $-(A)_p-$ Teil wird grundsätzlich jede reaktive Gruppe verstanden, die gegenüber einer Isocyanatgruppe inert bzw. reaktionsträge ist, beispielsweise die Isocyanatgruppe oder die Epoxygruppe. Eine hiermit coreaktive Gruppe einer oligofunktionellen Verbindung ist beispielsweise die Aminogruppe oder die Hydroxygruppe. Geeignete oligofunktionelle Verbindungen sind daher in diesem Fall z.B. Diamine, Diole oder Aminoalkohole. Weitere Beispiele sind dem Fachmann bekannt.

Die Gruppen, von denen, je nach Bedeutung des Index m, 1 bis 100 an das Makromer der Formel A gebunden sind, sind entweder endständig oder pendent oder endständig und pendent.

In einer besonders bevorzugten Ausführungsform hat das Makromer der Formel A zwei endständige Gruppen $R_xH$. Ein hieraus gebildetes erfindungsgemässes segmentiertes Copolymer der Formel D bzw. ein Blockcopolymer der Formel D ist ebenfalls besonders bevorzugt und wird in dieser Erfindung als Triblockcopolymer bezeichnet: Der zentrale Block wird im wesentlichen durch das Makromer gebildet, an das zwei Photoinitiatorrest-$(A)_p-R_a-$ Blöcke gebunden sind.

In einer anderen bevorzugten Ausführungsform weist das Makromer der Formel A nur pendente Gruppen $R_xH$ auf. Ein hieraus gebildetes erfindungsgemässes segmentiertes Copolymer der Formel D bzw. ein Pfropfcopolymer der Formel D ist ebenfalls bevorzugt und wird in dieser Erfindung als Kammpolymer bezeichnet: Der Rücken oder Steg des Kammes wird durch das Makromer gebildet, an das mehrere Photoinitiatoren pendent gebunden sind, die Zinken oder Zähne des Kammes werden im wesentlichen durch die bivalenten Reste A gebildet, die über den Rest des Photoinitiators gebunden sind.

In einer anderen bevorzugten Ausführungsform weist ein cylisches Makromer der Formel A pendente Gruppen $R_xH$ auf. Ein hieraus gebildetes erfindungsgemässes segmentiertes Copolymer der Formel D bzw. ein Pfropfcopolymer der Formel D ist ebenfalls bevorzugt und wird in dieser Erfindung als Sternpolymer bezeichnet: Der Mittelpunkt des Sterns wird durch das Makromer gebildet, an das mehrere Photoinitiatoren pendent gebunden sind, die Strahlen des Sternes werden im wesentlichen durch die bivalenten Reste A gebildet, die über den Rest des Photoinitiators gebunden sind.

Es ist bedeutsam, dass die erfindungsgemässen Vinyltelomere, die hieraus erhältlichen segmentierten Copolymere oder polymerisierbaren Verbindungen und die hieraus erhältlichen vernetzten Polymere sich in ihren Eigenschaften in überraschender Weise von herkömmlichen Komponenten unterscheiden. Dies gilt zum einen deshalb, weil sich die Kettenlänge der Vinylmonomere (siehe $-(A)_p-$ in Formel I) erfindungsgemäss weitgehend kontrollieren lässt, so dass z.B. eine vergleichsweise enge Molekulargewichtsverteilung erreicht werden kann. Ferner sind die Vinyltelomere der Formel I überraschenderweise frei oder zumindestens im wesentlichen frei von den Homopolymeren des jeweils verwendeten Vinylmonomeren sowie von Bis-OCN-terminierten Telomeren und Polymeren, die sonst üblicherweise durch Nebenreaktionen entstehen. Diese vorteilhaften Eigenschaften werden im Zuge der Herstellung der erfindungsgemässen polymerisierbaren Verbindungen der Formel C, der segmentierten Copolymere der Formel D und der

genannten Polymere auf diese übertragen.

Ferner weisen die erfindungsgemässen Vinyltelomere und die hieraus erhältlichen Produkte, wie sie z.B. vorstehend beschrieben sind, die Vorteile auf, dass sie durch eine einfache und schnelle Synthese mittels UV-Polymerisation zugänglich sind, dass bei ihrer Herstellung wenig Nebenreaktionen auftreten, dass die Produkte in aller Regel farblos sind und dass sie sich auch ausgehend von thermisch labilen Vinylmonomeren herstellen lassen. Es entstehen praktisch keine nicht mit der Isocyanatgruppe terminierten Vinyltelomere oder entsprechende Folgeprodukte durch eine etwaige Initiierung einer Homopolyerisation des Vinylmonomeren durch das zweite Radikalfragment des Photoinitators. Stattdessen zeigt das zweite, weniger reaktive Radikal offensichtlich während der Photopolymerisation einen gewissen Käfigeffekt und wirkt als effizienter Kettenabbrecher anstatt seinerseits eine Homopolymerisation des Vinylmonomeren zu starten. Auch wurden keine Nebenreaktionen beobachtet, die zu $\alpha,\omega$-difunktionellen Telomeren oder Polymeren führen.

Die erfindungsgemässen Copolymere der Formel D oder die polymerisierbaren Verbindungen der Formel C lassen sich gezielt zu Folgeprodukten umsetzen bzw weiterverarbeiten. Insbesondere hervorzuheben ist der Umstand, dass die unvernetzten Verbindungen der Formel C oder D sich auf einfache Weise in vernetzte Polymere einbauen lassen, z.B. dadurch dass die Umsetzung einer Verbindung der Formel C oder D in Gegenwart eines Vernetzers durchgeführt wird. Zusätzlich zu einer derartigen Vernetzung, oder alternativ dazu, können erfindungsgemässe Verbindungen der Formel C oder D modifiziert werden, wenn sie im Teil $-(A)_p-$ gemäss Formel I reaktive Gruppen aufweisen.

Bei derartigen reaktiven Gruppen kann es sich beispielsweise um Isocyanat- oder Epoxygruppen handeln, die von einem Vinylisocyanat oder einer Vinylepoxyverbindung stammen, wie beispielsweise von 2-Isocyanatoethylmethacrylat oder Glycidyl(meth)acrylat, die nachträglich mit einem Hydroxyniederalkyl(meth)acrylat, beispielsweise 2-Hydroxyethylmethacrylat oder 3-Hydroxypropylmethacrylat umgesetzt werden. Die C-C-Doppelbindungen eines Hydroxyniederalkyl(meth)acrylats, die auf die so beschriebene Weise eingebaut wurden, gestatten eine Vernetzung zu einem erfindungsgemässen Polymeren und / oder die Copolymerisation mit einem weiteren Vinylmonomeren oder Divinylmonomeren.

Vor- und nachstehend steht eine Formulierung "(meth)acrylat" abkürzend für "methacrylat oder acrylat".

Alle die vorstehend genannten Eigenschaften machen die erfindungsgemässen Polymere geeignet für eine Fülle von Einsatzzwecken als Formkörper verschiedener Art, wie als biomedizinische Materialien, z.B. Implantate, ophthalmische Linsen, insbesondere künstliche Hornhaut, intraokulare Linsen oder, ganz besonders bevorzugt, Kontaktlinsen, oder als medizinische Instrumente, Geräte, Membranen, Drug-Delivery Systeme, oder als Beschichtungen auf anorganischen oder organischen Materialien. Darüberhinaus sind die unvernetzten Vinyltelomere der Formel I nicht nur als Ausgangsmaterialien für die erfindungsgemässen Polymere geeignet, sondern auch hervorragend für die Herstellung von modifizierten Oberflächen geeignet, wie von biomedizinischen Materialien, ophthalmischen Linsen, insbesondere Kontaklinsen, oder Implantaten, sowie für Beschichtungen auf anorganischen oder organischen Materialien. Hierzu bedarf es nur der Umsetzung der hochreaktiven Isocyanatgruppe der Vinyltelomere der Formel I mit H-aktiven Gruppen der jeweiligen Oberflache. Mit hydrophilen Komponenten "A" entstehen amphiphile Block-, Kamm- oder Sternpolymere, die oberflächenaktive Eigenschaften besitzen und z.B. als Emulgatoren geeignet sind.

Die Erfindung betrifft daher unter anderem Formkörper, insbesondere Kontaktlinsen aus den genannten segmentierten Copolymeren, polymerisierbaren Verbindungen oder Polymeren. Die Erfindung betrifft ferner die Herstellung von Formkörpern, insbesondere von Kontaktlinsen, aus den genannten segmentierten Copolymeren, polymerisierbaren Verbindungen oder Polymeren, sowie die Verwendung der genannten segmentierten Copolymere, polymerisierbaren Verbindungen oder Polymere zur Herstellung von Formkörpern, insbesondere Kontaktlinsen, sowie die Verwendung der erfindungsgemässen Vinyltelomere zur Herstellung von modifizierten Oberflächen bzw. Beschichtungen, insbesondere der Oberflächen von Kontaktlinsen.

Bei den Makromeren der Formel A handelt es sich bevorzugt um Oligomere oder Polymere mit einem mittleren Molekulargewicht von 300 bis 10000 Dalton und sie enthalten bevorzugt mindestens 3, bevorzugter 3 bis 50 und besonders bevorzugt 5 bis 20 Struktureinheiten. Der Übergang zwischen Oligomeren und Polymeren ist bekannterweise fliessend und nicht genau abzugrenzen. Die Polymeren können 50 bis 10000, bevorzugter 50 bis 5 000 Struktureinheiten enthalten und ein mittleres Molekulargewicht von 10 000 bis 2 000 000, bevorzugt 10 000 bis 500 000 aufweisen. Die Oligomeren und Polymeren können auch bis zu 95 Mol-%, bevorzugter 5 bis 90 Mol-% comonomere Struktureinheiten ohne H-aktive Gruppen (dieser Terminus ist hier gleichbedeutend mit "$R_xH$ - Gruppen", die wie vorstehend definiert sind, mit der Massgabe, dass $R_x$ in diesem Fall von einer Bindung verschieden ist) enthalten, bezogen auf das Polymer.

Bei den Oligomeren und Polymeren mit H-aktiven Gruppen kann es sich um natürliche oder synthetische Oligomere oder Polymere handeln.

Natürliche Oligomere und Polymere sind zum Beispiel Oligo- und Polysaccharide oder deren Derivate, Proteine, Glycoproteine, Enzyme und Wachstumsfaktoren. Einige Beispiele sind Peptide, Cyclodextrine, Stärke, Hyaluronsäure, deacetylierte Hyaluronsäure, Chitosan, Trehalose, Cellobiose, Maltotriose, Maltohexaose, Chitohexaose, Agarose, Chitin 50, Amylose, Glucane, Heparin, Xylan, Pectin, Galactan, poly-Galactosamin, Glycosaminoglycane,

Dextran, aminiertes Dextran, Cellulose, Hydroxyalkylcellulosen, Carboxylalkylcellulosen, Heparin, Fucoidan, Chondroitinsulfat, sulfatierte Polysaccharide, Mucopolysaccharide, Gelatine, Zein, Casein, Seidenfibroin, Collagen, Albumin, Globulin, Bilirubin, Ovalbumin, Keratin, Fibronectin und Vitronectin, Pepsin, Trypsin und Lysozym.

Bei den synthetischen Oligomeren und Polymeren kann es sich um die Gruppen -COOH, -OH, $-NH_2$ oder -$NHR_N$ enthaltende Substanzen handeln, wobei $R_N$ bevorzugt $C_1$-$C_6$-Alkyl bedeutet. Es kann sich zum Beispiel um verseifte Polymerisate von Vinylestern oder -ethern (Polyvinylalkohol), hydroxylierte Polydiolefine wie z.B. Polybutadien, Polyisopren oder Chloropren; Polyacrylsäure und Polymethacrylsäure sowie Polyacrylate, Polymethacrylate, Polyacrylamide oder Polymethacrylamide mit Hydroxyalkyl- oder Aminoalkylresten in der Estergruppe oder Amidgruppe; Polysiloxane mit Hydroxyalkyl- oder Aminoalkylgruppen; Polyether aus Epoxiden oder Glycidylverbindungen und Diolen; Polyvinylphenole oder Copolymere von Vinylphenol und olefinischen Comonomeren; sowie Copolymerisate von mindestens einem Monomer aus der Gruppe Vinylalkohol, Vinylpyrrolidon, Acrylsäure, Methacrylsäure, (Meth)acrylsäureanhydrid oder Hydroxyalkyl oder Aminoalkyl enthaltenden Acrylaten, Methacrylaten, oder Acrylamid oder Methacrylamid, oder hydroxylierten Diolefinen mit ethylenisch ungesättigten Comonomeren wie z.B. Acrylnitril, Olefinen, Diolefinen, Vinylchlorid, Vinylidenchlorid, Vinylfluorid, Vinylidenfluorid, Styrol, $\alpha$-Methylstyrol, Vinylethern und Vinylestern; oder um Polyoxaalkylene mit endständigen OH- oder Aminoalkyloxygruppen handeln.

Bevorzugte Oligomere und Polymere sind zum Beispiel Cyclodextrine mit insgesamt 6 bis 8 einen Ring bildenden Glucosestruktureinheiten oder Hydroxyalkyl- oder Aminoalkylderivaten oder Glucose- oder Maltose-substituierten Derivaten, wovon mindestens eine Struktureinheit der Formel (V) entspricht,

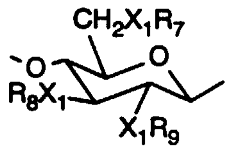

(V),

worin $R_7$, $R_8$ und $R_9$ unabhängig voneinander H, $C_1$-$C_4$-Alkyl, besonders Methyl, $C_2$-$C_6$-Acyl, besonders Acetyl, $C_1$-$C_4$-Hydroxyalkyl, besonders Hydroxymethyl oder 2-Hydroxyeth-1-yl, $C_2$-$C_{10}$-Aminoalkyl und besonders $C_2$-$C_4$-Aminoalkyl, zum Beispiel 2-Aminoeth-1-yl oder 3-Aminoprop-1-yl oder 4-Aminobut-1-yl bedeuten, $X_1$ für -O- oder -$NR_{1B}$-steht, wobei pro Cyclodextrineinheit insgesamt 1 bis 10 und bevorzugt 1 bis 6 $X_1$ -$NR_{1B}$-bedeuten können und die restlichen $X_1$ für -O- stehen, wobei $R_{1B}$ Wasserstoff oder Niederalkyl bedeutet.

Andere bevorzugte Oligomere und Polymere sind zum Beispiel Oligo- oder Polysiloxane mit OH- oder $NH_2$-Gruppen in Alkyl-, Alkoxyalkyl- oder Aminoalkylendgruppen oder -seitenketten. Es kann sich um statistische oder Blockoligomere oder Blockpolymere handeln. Bevorzugtere Oligomere und Polymere sind solche, die

a) 5 bis 100 Mol-% Strukturelemente der Formel (VII)

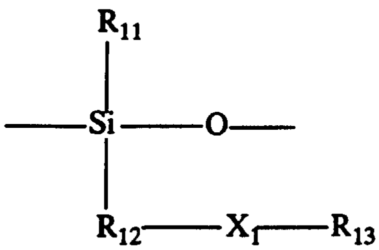

(VII),

und

b) 95 bis 0 Mol-% Strukturelemente der Formel (VIII)

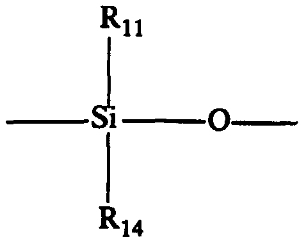

(VIII)

enthalten, bezogen auf das Oligomer oder Polymer, worin $R_{11}$ gegebenenfalls teilweise oder vollständig mit F substituiertes $C_1$-$C_4$-Alkyl, Niederalkenyl, Cyanniederalkyl oder Aryl, bevorzugt Methyl, Ethyl, Vinyl, Allyl, Cyanpropyl oder Trifluormethyl darstellt, $R_{12}$ $C_2$-$C_6$-Alkylen, bevorzugt 1,3-Propylen, $-(CH_2)_z-(O-CH_2-CHCH_3-)_z-$, $-(CH_2)_z-(O-CH_2-CH_2)_z-$ oder $-(CH_2)_z-NH-(CH_2)_z-NH-$, bevorzugt $-(CH_2)_3-(O-CH_2-CHCH_3)_2-$ oder $-(CH_2)_3-NH-(CH_2)_2-NH-$ bedeutet, wobei z eine ganze Zahl von 2 bis 4 darstellt, $R_{14}$ die Bedeutung von $R_{11}$ hat oder $-R_{12}-X_1-H$ oder $-R_{12}-X_1-R_{15}-H$ darstellt, $X_1$ für -O- oder -NH- steht, $R_{13}$ für einen Rest $R_xH$ steht, und $R_{15}$ eine direkte Bindung oder eine Gruppe $-C(O)-(CHOH)_r-CH_2-O-$ darstellt, worin r für 0 oder eine ganze Zahl von 1 bis 4 steht.

Bevorzugte oligomere oder polymere Siloxane sind auch solche der Formel (X)

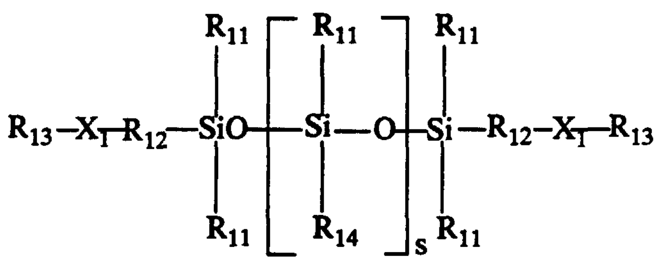

(X)

worin $R_{11}$ gegebenenfalls teilweise oder vollständig mit F substituiertes $C_1$-$C_4$-Alkyl, Vinyl, Allyl oder Phenyl, bevorzugt Methyl darstellt, $R_{12}$ $C_2$-$C_6$-Alkylen, bevorzugt 1,3-Propylen bedeutet, $R_{14}$ die Bedeutung von $R_{11}$ hat oder $-R_{12}-X_1-H$ oder $-R_{12}-X_1-R_{15}-H$ darstellt, $X_1$ für -O- oder -NH- steht, s für eine ganze Zahl von 1 - 1000 und bevorzugt von 1 - 150 steht, und $R_{13}$ für einen Rest $R_xH$ steht, und $R_{15}$ eine direkte Bindung oder eine Gruppe $-C(O)-(CHOH)_r-CH_2-O-$ darstellt, worin r für 0 oder eine ganze Zahl von 1 bis 4 steht. $X_1$ steht hier bevorzugt für -NH-.

Andere bevorzugte Oligomere und Polymere sind solche auf der Basis von Oligo- und Polyvinylalkohol. Es kann sich um Homopolymere mit $-CH_2CH(OH)-$Struktureinheiten oder um Copolymere mit anderen mono- oder bivalenten Struktureinheiten von Olefinen handeln.

Bevorzugter sind solche Oligomere und Polymere, welche

a) 5 bis 100 Mol-% Struktureinheiten der Formel (XI)

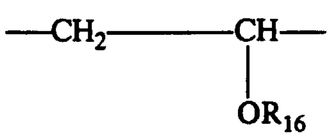

(XI),

und

b) 95 bis 0 Mol-% Struktureinheiten der Formel (XII)

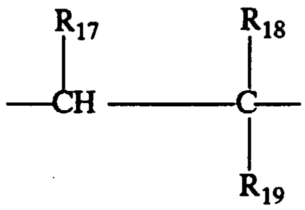

(XII)

enthalten, worin $R_{16}$ einen Rest $R_xH$ darstellt, $R_{17}$ für H, $C_1$-$C_6$-Alkyl, -$COOR_{20}$ oder -$COO^{\ominus}$ steht, $R_{18}$ H, F, Cl, CN oder $C_1$-$C_6$-Alkyl bedeutet, und $R_{19}$ H, OH, $R_{10}$-H, F, Cl, CN, $R_{20}$-O-, $C_1$-$C_{12}$-Alkyl, -$COO^{\ominus}$, -$COOR_{20}$, -OCO-$R_{20}$, Methylphenyl oder Phenyl darstellt, wobei $R_{10}$ eine direkte Bindung, -($C_1$-$C_4$-Alkylen-O)- oder -($C_2$-$C_{10}$-Alkylen-NH)- darstellt und $R_{20}$ für $C_1$-$C_{18}$-Alkyl, $C_5$-$C_7$-Cycloalkyl, ($C_1$-$C_{12}$-Alkyl)-$C_5$-$C_7$-cycloalkyl, Phenyl, ($C_1$-$C_{12}$-Alkyl)phenyl, Benzyl oder ($C_1$-$C_{12}$-Alkyl)benzyl steht.

$R_{17}$ steht bevorzugt für H. Bedeutet $R_{17}$ Alkyl, so handelt es sich bevorzugt um Methyl oder Ethyl. Bedeutet $R_{17}$ -$COOR_{20}$, so stellt $R_{20}$ bevorzugt $C_1$-$C_{12}$-, besonders $C_1$-$C_6$-Alkyl dar.

Bedeutet $R_{18}$ Alkyl, so handelt es sich bevorzugt um $C_1$-$C_4$-Alkyl, z.B. Methyl, Ethyl, n-Propyl oder n-Butyl. $R_{18}$ steht bevorzugt für H, Cl oder $C_1$-$C_4$-Alkyl.

Bedeutet $R_{19}$ die Gruppe $R_{20}$-O-, so stellt $R_{20}$ bevorzugt $C_1$-$C_{12}$-, besonders $C_1$-$C_6$-Alkyl dar. Bedeutet $R_{19}$ Alkyl, so enthält es bevorzugt 1 bis 6, besonders 1 bis 4 C-Atome. Bedeutet $R_{19}$ die Gruppe -$COOR_{20}$, so stellt $R_{20}$ bevorzugt $C_1$-$C_{12}$-, besonders $C_1$-$C_6$-Alkyl, Cyclopentyl oder Cyclohexyl dar. Bedeutet $R_{19}$ die Gruppe -OCO-$R_{20}$, so stellt $R_{20}$ bevorzugt $C_1$-$C_{12}$-, besonders $C_1$-$C_6$-Alkyl, Phenyl oder Benzyl dar.

In einer bevorzugten Ausführungsform stehen $R_{17}$ für H, $R_{18}$ für H, F, Cl, Methyl oder Ethyl, und $R_{19}$ für H, OH, F, Cl, CN, $C_1$-$C_4$-Alkyl, $C_1$-$C_6$-Alkoxy, $C_1$-$C_6$-Hydroxyalkoxy, -COO-$C_1$-$C_6$-Alkyl, -OOC-$C_1$-$C_6$-Alkyl oder Phenyl.

Besonders bevorzugt sind solche Oligomeren und Polymeren, worin $R_{17}$ H bedeutet, $R_{18}$ H oder Methyl darstellt, und $R_{19}$ H, OH, CN, Methyl, $OCH_3$, $O(CH_2)_tOH$ oder -$COOCH_3$ bedeutet, und t für ganze Zahlen von 2 bis 6 steht.

Eine weitere bevorzugte Gruppe von Oligomeren und Polymeren sind teilweise oder vollständig hydroxyalkylierte Oligo- oder Polyacrylate oder -methacrylate beziehungsweise -acrylamide oder -methacrylamide. Sie können zum Beispiel 5 bis 100 Mol-% Struktureinheiten der Formel (XIII)

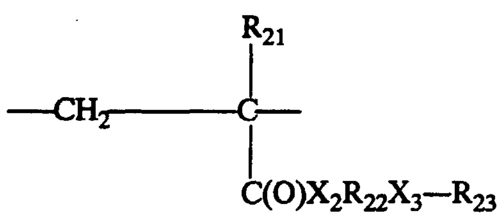

(XIII),

und 95 bis 0 Mol-% Struktureinheiten der Formel (XIV)

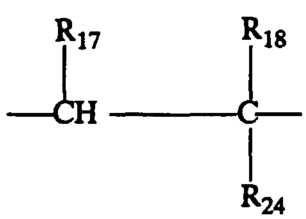

(XIV)

enthalten, worin $R_{21}$ H oder Methyl bedeutet, $X_2$ und $X_3$ unabhängig voneinander -O- oder -NH- darstellen, $R_{22}$ für -$(CH_2)_c$- steht und c eine ganze Zahl von 2 bis 12, vorzugsweise 2 bis 6 bedeutet, $R_{23}$ einen Rest der Formel $R_xH$ darstellt, $R_{17}$ und $R_{18}$ die zuvor angegebenen Bedeutungen haben, und $R_{24}$ die gleiche Bedeutung wie $R_{19}$ hat oder -$C(O)X_2R_{22}X_3H$ bedeutet. Für $R_{17}$, $R_{18}$ und $R_{19}$ gelten die zuvor angegebenen Bevorzugungen. Für $X_2$ und $X_3$ gelten die zuvor angegebenen Bevorzugungen.

Andere bevorzugte Oligomere und Polymere sind solche aus Polyalkylenoxiden. Es kann sich zum Beispiel um solche der Formel (XV) mit gleichen oder verschiedenen wiederkehrenden Struktureinheiten $-[CH_2CH(R_{26})-O]-$ handeln,

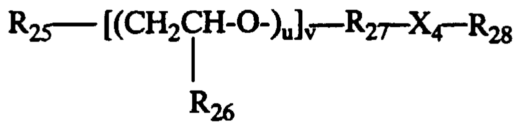

(XV),

worin $R_{25}$ die Gruppe $R_{28}$-$X_4$- darstellt oder der Rest eines Alkohols oder Polyols mit 1 bis 20 C-Atomen ist, wobei die Wertigkeit dieses Restes von 1 bis v beträgt, $R_{26}$ H, $C_1$-$C_8$-Alkyl, vorzugsweise $C_1$-$C_4$-Alkyl und insbesondere bevorzugt Methyl bedeutet, $R_{27}$ zusammen mit $X_4$ eine direkte Bindung oder $R_{27}$ $C_2$-$C_6$-Alkylen, vorzugsweise $C_3$-$C_6$-Alkylen und insbesondere bevorzugt 1,3-Propylen darstellt, $X_4$ für -O- oder -NH-steht, $R_{28}$ einen Rest der Formel $R_xH$ bedeutet, u für einen Zahlenwert von 3 bis 10 000, bevorzugt 5 bis 5 000, besonders bevorzugt 5 bis 1000 und insbesondere bevorzugt 5 bis 100 steht, und v eine ganze Zahl von 1 bis 6, bevorzugt 1 bis 4 bedeutet.

$R_{25}$ kann der ein- bis vierwertige Rest eines Alkohols oder Polyols sein. Wenn es sich bei $R_{25}$ um den Rest eines Alkohols handelt, so bedeutet $R_{25}$ vorzugsweise lineares oder verzweigtes $C_3$-$C_{20}$-Alkyl oder -Alkenyl, $C_3$-$C_8$- und besonders $C_5$-$C_6$-Cycloalkyl, $-CH_2-(C_5$-$C_6$-Cycloalkyl), $C_6$-$C_{10}$-Aryl und besonders Phenyl und Naphthyl, $C_7$-$C_{16}$-Aralkyl und besonders Benzyl und 1-Phenyleth-2-yl. Die cyclischen beziehungsweise aromatischen Reste können mit $C_1$-$C_{18}$-Alkyl oder $C_1$-$C_{18}$-Alkoxy substituiert sein.

Wenn es sich bei $R_{25}$ um den Rest eines Diols handelt, so bedeutet $R_{25}$ vorzugsweise verzweigtes und besonders lineares $C_3$-$C_{20}$-Alkylen oder Alkenylen und bevorzugter $C_3$-$C_{12}$-Alkylen, $C_3$-$C_8$- und besonders $C_5$-$C_6$-Cycloalkylen, $-CH_2-(C_5$-$C_6$-Cycloalkyl)-, $-CH_2-(C_5$-$C_6$-Cycloalkyl)$-CH_2-$, $C_7$-$C_{16}$-Aralkylen und besonders Benzylen, $-CH_2-(C_6$-$C_{10}$-Aryl)$-CH_2-$ und besonders Xylylen. Die cyclischen beziehungsweise aromatischen Reste können mit $C_1$-$C_{12}$-Alkyl oder $C_1$-$C_{12}$-Alkoxy substituiert sein.

Wenn es sich bei $R_{25}$ um einen dreiwertigen Rest handelt, so leitet sich dieser von aliphatischen oder aromatischen Triolen ab. $R_{25}$ ist bevorzugt ein dreiwertiger aliphatischer Rest mit 3 bis 12 C-Atomen, der sich besonders von Triolen mit vorzugsweise primären Hydroxylgruppen ableitet. Besonders bevorzugt stellt $R_{25}$ $-CH_2(CH-)CH_2-$, $HC(CH_2-)_3$ oder $CH_3C(CH_2-)_3$ dar.

Wenn es sich bei $R_{25}$ um einen vierwertigen Rest handelt, so leitet sich dieser bevorzugt von aliphatischen Tetrolen ab. $R_{25}$ ist in diesem Fall bevorzugt $C(CH_2-)_4$.

Bevorzugt steht $R_{25}$ für einen Rest, der abgeleitet ist von Jeffamine (Texaco), einem Pluriol, einem Poloxamer (BASF) oder Poly(tetramethylenoxid).

Besonders bevorzugt sind Homo- und Blockoligomere und -polymere mit Struktureinheiten der Formeln $-[CH_2CH_2-O]-$ oder $-[CH_2CH(CH_3)-O]-$.

Geeignet sind auch fluorierte Polyether, die der Formel (XVI)

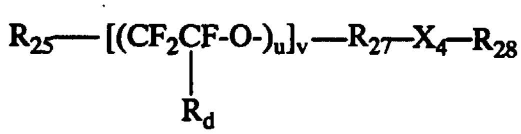

(XVI)

entsprechen, worin $R_{27}$, $R_{28}$, $X_4$, u und v die zuvor angegebenen Bedeutungen haben, $R_{25}$ die zuvor angegebene Bedeutung hat oder der einwertige Rest eines teil- oder perfluorierten Alkohols mit 1 bis 20, bevorzugt 1 bis 12 und besonders bevorzugt 1 bis 6 C-Atomen, oder der zweiwertige Rest eines teil- oder perfluorierten Diols mit 2 bis 6, bevorzugt 2 bis 4 und besonders bevorzugt 2 oder 3 C-Atomen ist, und $R_d$ F oder Perfluoralkyl mit 1 bis 12, bevorzugt 1 bis 6 und besonders bevorzugt 1 bis 4 C-Atomen bedeutet. $R_d$ steht besonders bevorzugt für $-CF_3$.

Weitere geeignete Oligomere und Polymere sind zum Beispiel Polyamine wie z.B. Polyvinylamin oder Polyethylenimine. Ebenfalls geeignet ist Poly-ε-lysin.

Als Photoinitiator der Formel B ist grundsätzlich jeder Photoinitiator geeignet, der eine Isocyanatgruppe enthält. Derartige Photoinitiatoren sind beispielsweise in der EP-A-632329 bereits beschrieben. Geeignete Photoinitiatoren weisen üblicherweise das Strukturelement

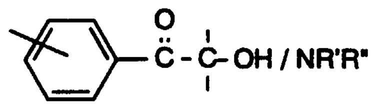

auf (wobei die Formulierung "OH / NR'R"" bedeutet, dass das fragliche Kohlenstoffatom entweder eine OH-Gruppe oder eine NR'R"-Gruppe trägt, worin R' und R" unabhängig voneinander lineares oder verzweigtes Niederalkyl, das mit $C_1$-$C_4$-Alkoxy substituiert sein kann, Arylniederalkyl oder Niederalkenyl bedeuten; oder R' und R" zusammen $-(CH_2)_z-Y_{11}-(CH_2)_z-$ bedeuten, wobei $Y_{11}$ eine direkte Bindung, -O-, -S-, oder $-NR_{1B}-$ ist und $R_{1B}$ H oder Niederalkyl bedeutet und z eine ganze Zahl von 2 bis 4 bedeutet), das bei geeigneter Anregung zwei Radikale bildet, indem die Bindung zwischen dem Benzoyl - Kohlenstoff und dem $sp^3$ - Kohlenstoff gespalten wird. Ueblicherweise ist das Benzoylradikal das reaktivere, das in aller Regel eine Polymerisation startet. Die Variable PI* aus Formel B entspricht daher vorzugsweise einem derartigen Benzoylradikal. Dieses Benzoylradikal ist, wie im Stand der Technik bekannt, substituiert und enthält erfindungsgemäss zusätzlich eine Isocyanatgruppe. Aus dem vorstehenden ergibt sich, dass das $sp^3$ - Kohlenstoffradikal das weniger reaktive ist, das in der Regel nicht dazu beiträgt, eine Polymerisation zu starten. Stattdessen reagiert es bevorzugt als Kettenabbrecher. Die Variable $R_{aa}$ aus Formel B entspricht daher vorzugsweise einem derartigen $sp^3$ - Kohlenstoffradikal.

Besonders bevorzugte erfindungsgemässe Photoinitiatoren werden nachstehend beschrieben.

Die erfindungsgemäss verwendeten funktionellen Photoinitiatoren der Formel B sind vorzugsweise Verbindungen der Formel IIa oder IIb

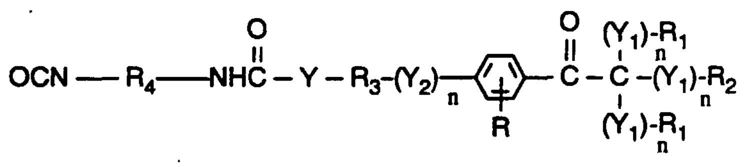

(IIa),

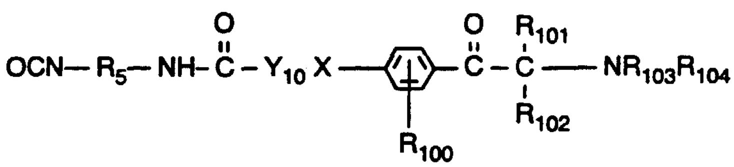

(IIb)

worin Y O, NH oder $NR_{1A}$ bedeutet; $Y_1$ O darstellt; $Y_2$ für -O-, -O-(O)C-, -C(O)-O- oder -O-C(O)-O- steht; die n unabhängig voneinander für 0 oder 1 stehen; R H, $C_1$-$C_{12}$-Alkyl, $C_1$-$C_{12}$-Alkoxy oder $C_1$-$C_{12}$-AlkylNH- darstellt; die $R_1$ und $R_2$ unabhängig voneinander H, lineares oder verzweigtes $C_1$-$C_8$-Alkyl, $C_1$-$C_8$-Hydroxyalkyl oder $C_6$-$C_{10}$-Aryl darstellen, oder zwei Gruppen $R_1$-$(Y_1)_n$- zusammen $-(CH_2)_x-$ bedeuten, oder die Gruppen $R_1$-$(Y_1)_n$- und $R_2$-$(Y_1)_n$- zusammen einen Rest der Formel

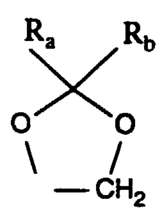

bilden; $R_3$ eine direkte Bindung oder lineares oder verzweigtes $C_1$-$C_8$-Alkylen darstellt, das unsubstituiert oder mit -OH substituiert ist und/oder gegebenenfalls mit ein oder mehreren Gruppen -O-, -O-C(O)- oder -O-C(O)-O- unterbrochen ist; $R_4$ verzweigtes $C_3$-$C_{18}$-Alkylen, unsubstituiertes oder mit $C_1$-$C_4$-Alkyl oder $C_1$-$C_4$-Alkoxy substituiertes $C_6$-$C_{10}$-Arylen, oder unsubstituiertes oder mit $C_1$-$C_4$-Alkyl oder $C_1$-$C_4$-Alkoxy substituiertes $C_7$-$C_{18}$-Aralkylen, unsubstituiertes oder mit $C_1$-$C_4$-Alkyl oder $C_1$-$C_4$-Alkoxy substituiertes $C_3$-$C_8$-Cycloalkylen, unsubstituiertes oder mit $C_1$-$C_4$-Alkyl oder $C_1$-$C_4$-Alkoxy substituiertes $C_3$-$C_8$-Cycloalkylen-$C_yH_{2y}$- oder unsubstituiertes oder mit $C_1$-$C_4$-Alkyl oder $C_1$-$C_4$-Alkoxy

substituiertes -$C_yH_{2y}$-($C_3$-$C_8$-Cycloalkylen)-$C_yH_{2y}$- bedeutet; $R_5$ unabhängig die gleiche Bedeutung wie $R_4$ hat oder lineares $C_3$-$C_{18}$-Alkylen darstellt; $R_{1A}$ für Niederalkyl steht; x ganze Zahlen von 3 bis 5 bedeutet; y ganze Zahlen von 1 bis 6 bedeutet; $R_a$ und $R_b$ unabhängig voneinander H, $C_1$-$C_8$-Alkyl, $C_3$-$C_8$-Cycloalkyl, Benzyl oder Phenyl darstellen; mit den Massgaben, dass n in den Gruppen -$(Y_1)_n$-$R_1$ für 0 steht, wenn $R_2$ H bedeutet; dass höchstens zwei $Y_1$ der -$(Y_1)_n$-Gruppen O bedeuten sowie n in den anderen -$(Y_1)_n$-Gruppen für 0 steht; und dass n in der Gruppe -$(Y_2)_n$- für 0 steht, wenn $R_3$ eine direkte Bindung bedeutet; und worin ferner X bivalentes -O-, -NH-, -S-, Niederalkylen oder

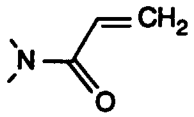

bedeutet; $Y_{10}$ -O-$(CH_2)_y$-oder eine direkte Bindung darstellt, wobei y ganze den von 1 - 6 bedeutet und dessen endständige $CH_2$-Gruppe mit dem benachbarten X in Formel (IIb) verknüpft ist; $R_{100}$ H, $C_1$-$C_{12}$-Alkyl, $C_1$-$C_{12}$-Alkoxy, $C_1$-$C_{12}$-AlkylNH- oder -$NR_{1A}R_{1B}$ darstellt, wobei $R_{1A}$ für Niederalkyl und $R_{1B}$ für H oder Niederalkyl steht; $R_{101}$ für lineares oder verzweigtes Niederalkyl, Niederalkenyl oder Arylniederalkyl steht; $R_{102}$ unabhängig von $R_{101}$ die gleiche Bedeutung wie $R_{101}$ hat oder Aryl bedeutet, oder $R_{101}$ und $R_{102}$ zusammen -$(CH_2)_m$- bedeuten, wobei m ganze den von 2 - 6 bedeutet; $R_{103}$ und $R_{104}$ unabhängig voneinander lineares oder verzweigtes Niederalkyl, das mit $C_1$-$C_4$-Alkoxy substituiert sein kann, Arylniederalkyl oder Niederalkenyl bedeuten; oder $R_{103}$ und $R_{104}$ zusammen -$(CH_2)_z$-$Y_{11}$-$(CH_2)_z$- bedeuten, wobei $Y_{11}$ eine direkte Bindung, -O-, -S-, oder $NR_{1B}$- ist und $R_{1B}$ H oder Niederalkyl bedeutet und z eine ganze Zahl von 2 bis 4 bedeutet.

In einer bevorzugten Ausführungsform steht Y für O.

$R_{1A}$ als Alkyl kann zum Beispiel Methyl, Ethyl, n- oder i-Propyl, n-, i- oder t-Butyl, Pentyl oder Hexyl sein. Bevorzugt stellt $R_{1A}$ Methyl dar.

Die Gruppe R enthält als Alkyl, Alkoxy oder AlkylNH- bevorzugt 1 bis 6 und besonders bevorzugt 1 bis 4 C-Atome. Einige Beispiele sind Methyl, Ethyl, n- oder i-Propyl, n-, i-oder t-Butyl, Pentyl, Hexyl, Octyl, Decyl, Dodecyl, Methoxy, Ethoxy, Propoxy, Butoxy, und MethylNH-. Insbesondere bevorzugt steht R für H.

$R_1$ ist als Alkyl bevorzugt linear und enthält bevorzugt 1 bis 4 C-Atome. Einige Beispiele sind Methyl, Ethyl, n- oder i-Propyl, n-, i- oder t-Butyl, Pentyl, Hexyl, Heptyl und Octyl. Besonders bevorzugt handelt es sich bei $R_1$ um Methyl oder Ethyl. $R_1$ kann als Aryl zum Beispiel Naphthyl und besonders Phenyl bedeuten. Wenn beide Gruppen $R_1$-$(Y_1)_n$- zusammen für -$(CH_2)_x$- stehen, ist x bevorzugt 4 und besonders bevorzugt 5. $R_1$ ist als Hydroxyalkyl bevorzugt linear und enthält bevorzugt 1 bis 4 C-Atome. Einige Beispiele sind Hydroxymethyl und 2-Hydroxyeth-1-yl.

Für $R_2$ gelten die gleichen Bevorzugungen wie für $R_1$. Besonders bevorzugt steht $R_2$ für H, Methyl oder Ethyl.

$R_a$ und $R_b$ bedeuten bevorzugt unabhängig voneinander H oder $C_1$-$C_4$-Alkyl, zum Beispiel Methyl oder Ethyl.

In einer bevorzugten Untergruppe bedeutet $R_1$ bevorzugt Ethyl und besonders bevorzugt Methyl oder die beiden Gruppen $R_1$-$(Y_1)_n$- zusammen Pentamethylen, steht n in der Gruppe -$(Y_1)_n$-$R_2$ bevorzugt für 0, stellt $R_2$ bevorzugt Methyl, Hydroxmethyl oder H dar und steht R für H.

In einer anderen bevorzugten Ausführungsform stehen in der Gruppe -$(Y_1)_n$-$R_2$ $Y_1$ für O, n für 1 und $R_2$ für H. Insbesondere steht in diesem Fall n in den Gruppen $R_1$-$(Y_1)_n$- für 0.

$R_3$ enthält als Alkylen bevorzugt 1 bis 6 und besonders bevorzugt 1 bis 4 C-Atome und bevorzugt ist das Alkylen linear. Einige Beispiele sind Methylen, Ethylen, 1,2- oder 1,3-Propylen, 1,2-, 1,3- oder 1,4-Butylen, Pentylen, Hexylen, Heptylen und Octylen. Bevorzugt sind Methylen, Ethylen, 1,3-Propylen und 1,4-Butylen. Ganz besonders bevorzugt stellt $R_3$ Ethylen dar; oder eine direkte Bindung, wobei n in der Gruppe -$(Y_2)_n$- für 0 steht.

Bei $R_3$ als mit Hydroxy substituiertem Alkylen kann es sich zum Beispiel insbesondere um 2-Hydroxy-1,3-propylen oder auch um 2-Hydroxy-1,3- oder-1,4-butylen handeln. Mit -O- unterbrochenes und gegebenenfalls mit -OH substituiertes Alkylen ist zum Beispiel -$CH_2CH_2$-O-$CH_2CH_2$-, -$CH_2CH_2$-O-$CH_2CH_2$-O-$CH_2CH_2$-, -$CH_2CH_2$-O-$CH_2CH_2$-O-$CH_2CH_2$-O-$CH_2CH_2$-, [-$CH(CH_3)CH_2$-O-$CH(CH_3)CH_2$-], -$CH(CH_3)CH_2$-O-$CH_2CH_2$-, -$CH(C_2H_5)CH_2$-O-$CH_2CH_2$-, [-$CH(C_2H_5)CH_2$-O-$CH(C_2H_5)CH_2$-] oder -$CH_2CH_2CH_2CH_2$-O-$CH_2CH_2CH_2CH_2$- und -$CH_2CH(OH)CH_2$-O-$CH_2CH_2$-. Mit -O-C(O)-oder -C(O)-O- unterbrochenes Alkylen ist zum Beispiel -$CH_2CH_2$-C(O)-O-$CH_2$- oder -$CH_2CH_2$-O-C(O)-$CH_2$-. Mit -O-C(O)-O- unterbrochenes Alkylen ist zum Beispiel -$CH_2CH_2$-O-C(O)-O-$CH_2CH_2$- oder -$CH_2CH_2$-O-C(O)-O-$CH_2$-.

Bei den Substituenten $C_1$-$C_4$-Alkyl und $C_1$-$C_4$-Alkoxy handelt es sich vorzugsweise um Methyl, Ethyl, Methoxy oder Ethoxy.

$R_4$ enthält als verzweigtes Alkylen bevorzugt 3 bis 14 und besonders bevorzugt 4 bis 10 C-Atome. Beispiele für Alkylen sind 1,2-Propylen, 2-Methyl- oder 2,2-Dimethyl-1,3-propylen, 1,2-, 1,3- und 2,3-Butylen, 2-Methyl- oder 2,3-Dimethyl-1,4-butylen, 1,2-, 1,3- oder 1,4-Pentylen, 2-Methyl- oder 3-Methyl- oder 4-Methyl- oder 2,3-Dimethyl- oder 2,4-Dimethyl oder 3,4-Dimethyl- oder 2,3,4-Trimethyl oder 2,2,3-Trimethyl- 2,2,4-Trimethyl- oder 2,2,3,3-Tetramethyl- oder 2,2,3,4-Tetramethyl-1,5-pentylen, 1,2-, 1,3-, 1,4- oder 1,5-Hexylen, 2-Methyl- oder 3-Methyl oder 4-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl- oder 2,2,4-Trimethyl- oder 2,2,5-Trimethyl- oder 2,3,4-Trimethyl- oder 2,2,4,5-Tetramethyl-1,6-hexylen. Weitere Beispiele sind in der EP-A-632329 offenbart.

Einige bevorzugte verzweigte Alkylenreste sind 2,2-Dimethyl-1,4-butylen, 2,2-Dimethyl-1,5-pentylen, 2,2,3- oder 2,2,4-trimethyl-1,5-pentylen, 2,2-Dimethyl-1,6-hexylen, 2,2,3- oder 2,2,4- oder 2,2,5-Trimethyl-1,6-hexylen, 2,2-Dimethyl-1,7-heptylen, 2,2,3- oder 2,2,4- oder 2,2,5- oder 2,2,6-Trimethyl-1,7-heptylen, 2,2-Dimethyl-1,8-octylen, 2,2,3- oder 2,2,4- oder 2,2,5- oder 2,2,6- oder 2,2,7-Trimethyl-1,8-octylen.

Wenn $R_4$ Arylen ist, handelt es sich bevorzugt um Naphthylen und besonders bevorzugt um Phenylen. Wenn das Arylen substituiert ist, befindet sich ein Substituent vorzugsweise in Orthostellung zu einer Isocyanatgruppe. Beispiele für substituiertes Arylen sind 1-Methyl-2,4-phenylen, 1,5-Dimethyl-2,4-phenylen, 1-Methoxy-2,4-phenylen und 1-Methyl-2,7-naphthylen.

$R_4$ als Aralkylen ist bevorzugt Naphthylalkylen und besonders bevorzugt Phenylalkylen. Die Alkylengruppe im Aralkylen enthält bevorzugt 1 bis 12, besonders bevorzugt 1 bis 6 und insbesondere bevorzugt 1 bis 4 C-Atome. Ganz besonders bevorzugt stellt die Alkylengruppe rm Aralkylen Methylen oder Ethylen dar. Einige Beispiele sind 1,3- oder 1,4-Benzylen, Naphth-2-yl-7-methylen, 6-Methyl-1,3- oder 1,4-benzylen, 6-Methoxy-1,3- oder 1,4-benzylen.

Wenn $R_4$ Cycloalkylen ist, handelt es sich bevorzugt um $C_5$- oder $C_6$-Cycloalkylen, das unsubstituiert oder mit Methyl substituiert ist. Einige Beispiele sind 1,3-Cyclobutylen, 1,3-Cyclopentylen, 1,3- oder 1,4-Cyclohexylen, 1,3- oder 1,4-Cycloheptylen, 1,3- oder 1,4- oder 1,5-Cyclooctylen, 4-Methyl-1,3-Cyclopentylen, 4-Methyl-1,3-Cyclohexylen, 4,4-Dimethyl-1,3-Cyclohexylen, 3-Methyl- oder 3,3-Dimethyl-1,4-Cyclohexylen, 3,5-Dimethyl-1,3-Cyclohexylen, 2,4-Dimethyl-1,4-Cyclohexylen.

Wenn $R_4$ Cycloalkylen-$C_yH_{2y}$- bedeutet, handelt es sich bevorzugt um Cyclopentylen-$C_yH_{2y}$- und besonders um Cyclohexylen-$C_yH_{2y}$-, das unsubstituiert oder mit vorzugsweise 1 bis 3 $C_1$-$C_4$-Alkyl, besonders bevorzugt Methyl, substituiert ist. In der Gruppe -$C_yH_{2y}$- steht y bevorzugt für ganze Zahlen von 1 bis 4. Bevorzugter stellt die Gruppe -$C_yH_{2y}$- Ethylen und besonders bevorzugt Methylen dar. Einige Beispiele sind Cyclopent-1-yl-3-methylen, 3-Methyl-cyclopent-1-yl-3-methylen, 3,4-Dimethyl-cyclopent-1-yl-3-methylen, 3,4,4-Trimethyl-cyclopent-1-yl-3-methylen, Cyclohex-1-yl-3- oder -4-methylen, 3- oder 4 -oder 5-Methyl-cyclohex-1-yl-3- oder -4-methylen, 3,4- oder 3,5-Dimethyl-cyclohex-1-yl-3- oder -4-methylen, 3,4,5 -oder 3,4,4- oder 3,5,5-Trimethyl-cyclohex-1-yl-3- oder -4-methylen.

Wenn $R_4$ -$C_yH_{2y}$-Cycloalkylen-$C_yH_{2y}$- bedeutet, handelt es sich bevorzugt um -$C_yH_{2y}$-Cydopentylen-$C_yH_{2y}$- und besonders um -$C_yH_{2y}$-Cyclohexylen-$C_yH_{2y}$-, das unsubstituiert oder mit vorzugsweise 1 bis 3 $C_1$-$C_4$-Alkyl, besonders bevorzugt Methyl, substituiert ist. In der Gruppe -$C_yH_{2y}$- steht y bevorzugt für ganze Zahlen von 1 bis 4. Bevorzugter stellen die Gruppen -$C_yH_{2y}$- Ethylen und besonders bevorzugt Methylen dar. Einige Beispiele sind Cyclopentan-1,3-dimethylen, 3-Methyl-cyclopentan-1,3-dimethylen, 3,4-Dimethyl-cyclopentan-1,3-dimethylen, 3,4,4-Trimethyl-cyclopentan-1,3-dimethylen, Cyclohexan-1,3- oder -1,4-dimethylen, 3- oder 4- oder 5-Methyl-cyclohexan-1,3- oder -1,4-dimethylen, 3,4- oder 3,5-Dimethyl-cyclohexan-1,3- oder -1,4-dimethylen, 3,4,5- oder 3,4,4-oder 3,5,5-Trimethyl-cyclohexan-1,3- oder -1,4-dimethylen.

Wenn $R_5$ die gleiche Bedeutung wie $R_4$ hat, gelten auch die zuvor für $R_4$ angegebenen Bevorzugungen. $R_5$ enthält als lineares Alkylen bevorzugt 3 bis 12 und besonders bevorzugt 3 bis 8 C-Atome. Einige Beispiele für lineares Alkylen sind 1,3-Propylen, 1,4-Butylen, 1,5-Pentylen, 1,6-Hexylen, 1,7-Heptylen, 1,8-Octylen, 1,9-Nonylen, 1,10-Decylen, 1,11-Undecylen, 1,12-Dodecylen, 1,14-Tetradecylen und 1,18-Octadecylen.

Eine bevorzugte Bedeutung von X ist -O-, -NH-, -S- oder Niederalkylen. Stärker bevorzugt steht X für -O- oder -S- und besonders bevorzugt für -O-.

In einer bevorzugten Bedeutung von $Y_{10}$ steht der Index y für 1 - 5, stärker bevorzugt für 2 - 4 und ausserordentlich bevorzugt für 2 - 3, so dass $Y_{10}$ zum Beispiel Ethylenoxy oder Propylenoxy bedeutet. In einer weiteren bevorzugten Bedeutung steht $Y_{10}$ für eine direkte Bindung, wobei X dann bevorzugt mindestens ein Heteroatom darstellt oder enthält.

Die Gruppe $R_{100}$ enthält als Alkyl, Alkoxy, AlkylNH- oder -$NR_{1A}R_{1B}$ bevorzugt 1 bis 6 und besonders bevorzugt 1 bis 4 C-Atome. Einige Beispiele sind Methyl, Ethyl, n- oder i-Propyl, n-, i- oder t-Butyl, Pentyl, Hexyl, Octyl, Decyl, Dodecyl, Methoxy, Ethoxy, Propoxy, Butoxy, N,N-Dimethylamino und N-Methylamino. Insbesondere bevorzugt steht R für H. Eine bevorzugte Bedeutung von -$NR_{1A}R_{1B}$ ist N,N-Dimethylamino, N-Methylamino, N-Methyl-N-Ethylamino, N-Ethylamino, N,N-Diethylamino, N-Isopropylamino oder N,N-Diisopropylamino.

$R_{101}$ bedeutet bevorzugt Allyl, Benzyl, lineares $C_1$-$C_4$-Alkyl wie zum Beispiel Methyl oder Ethyl.

$R_{102}$ hat bevorzugt die gleiche Bedeutung wie $R_{101}$, ist stärker bevorzugt lineares Niederalkyl mit 1 bis 4 C-Atomen und besonders bevorzugt 1 bis 2 C-Atomen. $R_{102}$ kann als Aryl zum Beispiel Naphthyl oder besonders Phenyl bedeuten, das unsubstituiert oder durch Niederalkyl oder Niederalkoxy substituiert ist. Wenn $R_{101}$ und $R_{102}$ zusammen für -$(CH_2)_m$- stehen, ist m bevorzugt 4 oder 5 und besonders bevorzugt 5.

$R_{103}$ steht bevorzugt für lineares Niederalkyl mit 1 bis 4 C-Atomen, Benzyl oder Allyl, und stärker bevorzugt für Methyl oder Ethyl.

$R_{104}$ steht bevorzugt für lineares Niederalkyl mit 1 bis 4 C-Atomen, und stärker bevorzugt für Methyl oder Ethyl.

Wenn $R_{103}$ und $R_{104}$ zusammen -$(CH_2)_z$-$Y_{11}$-$(CH_2)_z$- bedeuten ist $Y_{11}$ bevorzugt eine direkte Bindung, -O-

oder $-N(CH_3)-$ und ganz besonders -O-; z ist bevorzugt 2 - 3 und besonders bevorzugt 2.

Eine bevorzugte Untergruppe von Verbindungen der Formel IIa sind solche, worin in den Gruppen $R_1-(Y_1)_n-$ n für 0 steht, Y, $Y_2$ und $Y_1$ in der Gruppe $R_2-(Y_1)_n-$ je O bedeuten, n in der Gruppe $R_2-(Y_1)_n-$ für 0 oder 1 steht, $R_1$ $C_1$-$C_4$-Alkyl oder Phenyl bedeuten oder die Gruppen $R_1-(Y_1)_n-$ zusammen Tetramethylen oder Pentamethylen darstellen, $R_2$ $C_1$-$C_4$-Alkyl oder H darstellt, R Wasserstoff bedeutet, n in der Gruppe $-(Y_2)-_n$ für 0 oder 1 steht, $R_3$ lineares oder verzweigtes $C_2$-$C_4$-Alkylen darstellt oder eine direkte Bindung bedeutet, wobei n in der Gruppe $-(Y_2)-_n$ für 0 steht, $R_4$ verzweigtes $C_5$-$C_{10}$-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexyl-$C_yH_{2y}$- oder -$C_yH_{2y}$-Cyclohexyl-$C_yH_{2y}$- oder mit 1 bis 3 Methylgruppen substituiertes Cyclohexyl-$C_yH_{2y}$- oder -$C_yH_{2y}$-Cyclohexyl-$C_yH_{2y}$-bedeutet, $R_5$ die für $R_4$ angegebenen Bedeutungen hat oder lineares $C_3$-$C_{10}$-Alkylen darstellt, und y für 1 oder 2 steht.

Eine besonders bevorzugte Untergruppe von Verbindungen der Formel IIa sind solche, worin in den Gruppen $R_1-(Y_1)_n-$ und $-(Y_2)-_n$ n für 0 steht, Y, $Y_2$ und $Y_1$ in der Gruppe $R_2-(Y_1)_n-$ je O bedeuten, n in der Gruppe $R_2-(Y_1)_n-$ für 0 oder 1 steht, $R_1$ Methyl oder Phenyl bedeutet oder die Gruppen $R-(Y_1)_n-$ zusammen Pentamethylen darstellen, $R_2$ Methyl oder H darstellt, R Wasserstoff bedeutet, n in der Gruppe $-(Y_2)-_n$ für 1 steht und $R_3$ Ethylen darstellt oder n in der Gruppe $-(Y_2)-_n$ für 0 steht und $R_3$ eine direkte Bindung bedeutet, $R_4$ verzweigtes $C_6$-$C_{10}$-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexyl-$CH_2$- oder mit 1 bis 3 Methylgruppen substituiertes Cyclohexyl-$CH_2$ bedeutet, und $R_5$ die für $R_4$ angegebenen Bedeutungen hat oder lineares $C_5$-$C_{10}$-Alkylen darstellt.

Eine bevorzugte Untergruppe von Verbindungen der Formel IIb sind solche, worin $R_{101}$ für lineares Niederalkyl, Niederalkenyl oder Arylniederalkyl steht; $R_{102}$ unabhängig von $R_{101}$ die gleiche Bedeutung wie $R_{101}$ hat oder Aryl bedeutet; $R_{103}$ und $R_{104}$ unabhängig voneinander lineares oder verzweigtes Niederalkyl, das mit $C_1$-$C_4$-Alkoxy substituiert sein kann, Arylniederalkyl oder Niederalkenyl bedeuten; oder $R_{103}$ und $R_{104}$ zusammen $-(CH_2)_z-Y_{11}-(CH_2)_z-$ bedeuten, wobei $Y_{11}$ eine direkte Bindung, -O-, -S-, oder $NR_{1B}$- ist und $R_{1B}$ H oder Niederalkyl bedeutet und z eine ganze Zahl von 2 bis 4 bedeutet; und $R_5$ lineares oder verzweigtes $C_3$-$C_{18}$-Alkylen, unsubstituiertes oder mit $C_1$-$C_4$-Alkyl oder $C_1$-$C_4$-Alkoxy substituiertes $C_6$-$C_{10}$-Arylen, oder unsubstituiertes oder mit $C_1$-$C_4$-Alkyl oder $C_1$-$C_4$-Alkoxy substituiertes $C_7$-$C_{18}$-Aralkylen, unsubstituiertes oder mit $C_1$-$C_4$-Alkyl oder $C_1$-$C_4$-Alkoxy substiuiertes $C_{13}$-$C_{24}$-Arylenalkylenarylen, unsubstituiertes oder mit $C_1$-$C_4$-Alkyl oder $C_1$-$C_4$-Alkoxy substituiertes $C_3$-$C_8$-Cycloalkylen, unsubstituiertes oder mit $C_1$-$C_4$-Alkyl oder $C_1$-$C_4$-Alkoxy substituiertes $C_3$-$C_8$-Cycloalkylen-$C_yH_{2y}$- oder unsubstituiertes oder mit $C_1$-$C_4$-Alkyl oder $C_1$-$C_4$-Alkoxy substituiertes -$C_yH_{2y}$-($C_3$-$C_8$-Cycloalkylen)-$C_yH_{2y}$- bedeutet, wobei y eine ganze Zahl von 1 - 6 bedeutet.

Eine bevorzugte Untergruppe von Verbindungen der Formel IIb sind solche, worin X bivalentes -O-, -NH-, -S- oder $-(CH_2)_y-$ bedeutet; $Y_{10}$-O-$(CH_2)_y$- oder eine Bindung darstellt, wobei y ganze Zahlen von 1 - 6 bedeutet und dessen endständige $CH_2$-Gruppe mit dem benachbarten X in Formel (IIb) verknüpft ist; $R_{100}$ H, $C_1$-$C_{12}$-Alkyl oder $C_1$-$C_{12}$-Alkoxy darstellt; $R_{101}$ für lineares Niederalkyl, Niederalkenyl oder Arylniederalkyl steht; $R_{102}$ unabhängig von $R_{101}$ die gleiche Bedeutung wie $R_{101}$ hat oder Aryl bedeutet, oder $R_{101}$ und $R_{102}$ zusammen $-(CH_2)_m$ bedeuten, wobei m ganze den von 2 - 6 bedeutet; $R_{103}$ und $R_{104}$ unabhängig voneinander lineares oder verzweigtes Niederalkyl, da mit $C_1$-$C_4$-Alkoxy substituiert sein kann, Arylniederalkyl oder Niederalkenyl bedeuten; oder $R_{103}$ und $R_{104}$ zusammen $-(CH_2)_z-Y_{11}-(CH_2)_z-$ bedeuten, wobei $Y_{11}$ eine direkte Bindung, -O-, -S-, oder $-NR_{1B}-$ ist und $R_{1B}$ H oder Niederalkyl bedeutet und z eine ganze Zahl von 2 bis 4 bedeutet; und $R_5$ verzweigtes $C_6$-$C_{10}$-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexylen-$CH_2$- oder mit 1 bis 3 Methylgruppen substituiertes Cyclohexylen-$CH_2$- bedeutet.

Eine besonders bevorzugte Untergruppe von Verbindungen der Formel IIb sind solche, worin $R_{101}$ Methyl, Allyl, Toluylmethyl oder Benzyl bedeutet, $R_{102}$ Methyl, Ethyl, Benzyl oder Phenyl bedeutet oder $R_{101}$ und $R_{102}$ zusammen Pentamethylen darstellen, $R_{103}$ und $R_{104}$ unabhängig voneinander für Niederalkyl mit bis zu 4 C-Atomen stehen oder $R_{103}$ und $R_{104}$ zusammen für $-CH_2CH_2OCH_2CH_2-$ stehen, und $R_5$ verzweigtes $C_6$-$C_{10}$-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cydohexylen-$CH_2$- oder mit 1 bis 3 Methylgruppen substituiertes Cyclohexylen-$CH_2$- bedeutet.

Bei den Gruppen $R_4$ und $R_5$ handelt es sich insbesondere um solche, die die Reaktivität der OCN-Gruppe vermindern, was im wesentlichen durch eine sterische Hinderung oder elektronische Einflüsse an mindestens einem benachbarten C-Atom erreicht wird. Bevorzugt sind $R_4$ und $R_5$ daher unter anderem unsymmetrische Reste, z.B. in $\alpha$- oder besonders $\beta$-Stellung zur OCN-Gruppe verzweigtes Alkylen, oder in mindestens einer $\alpha$-Stellung wie definiert substituierte cyclische Kohlenwasserstoffreste.

Unter einem copolymerisierbaren Vinylmonomer wird im Rahmen dieser Erfindung insbesondere ein Monomer verstanden, das eine Vinylgruppe enthält und bereits im Zusammenhang mit Copolymerisaten, die für Kontaktlinsen Verwendung gefunden haben, erwähnt wurde. Unter einer Vinylgruppe wird in diesem Zusammenhang nicht ausschliesslich die Vinylgruppierung "$-CH=CH_2$" verstanden, sondern allgemein jede Gruppierung, die eine Kohlenstoff-Kohlenstoff-Doppelbindung aufweist. Speziell bevorzugte Bedeutungen des Wortbestandteils "Vinyl" bei Vinylmonomeren werden aus den nachstehenden Erläuterungen im Zusammenhang mit Verbindungen der Formel III deutlich. Copolymerisierbare Vinylmonomere im Sinne dieser Erfindung sind beispielsweise in den EP-A-374,752, EP-A-417,235 und in der EP-A-455,587 bereits offenbart worden.

Insbesondere sind die Monomere, von denen man ausgeht, um den Bestandteil A der Formel I für die erfindungsgemässen Vinyltelomere, Blockcopolymere, polymerisierbaren Verbindungen, Polymere oder Kontaktlinsen bereitzustellen, Verbindungen der Formel III,

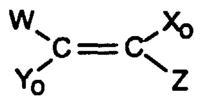

(III)

die, symbolisiert durch den Buchstaben A, in das Vinyltelomere der Formel I in Gestalt der Teilformel IV eingebaut werden,

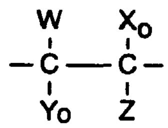

(IV)

wobei die Substituenten W, $X_o$, $Y_o$ und Z die folgenden Bedeutungen aufweisen: drei dieser Substituenten bedeuten Wasserstoff und der vierte Substituent ist ausgewählt unter Acyl, Halogen, einem heterocyclischen Rest oder Aryl, oder zwei dieser Substituenten bedeuten Wasserstoff, der dritte bedeutet Niederalkyl, und der vierte Substituent ist ausgewählt unter Acyl, Halogen, einem heterocyclischen Rest oder Aryl, oder zwei dieser Substituenten bedeuten Wasserstoff und die beiden anderen Substituenten bilden gemeinsam eine Kohlenwasserstoffbrücke, die ununterbrochen oder durch ein oder zwei Heteroatome unterbrochen ist, oder die beiden anderen Substituenten bedeuten unabhängig voneinander Acyl. Die Monomere der Formel III sind entweder hydrophile Vinylmonomere oder hydrophobe Vinylmonomere.

Vorzugsweise weisen diejenigen copolymerisierbaren Vinylmonomere, die zur Herstellung von Vinyltelomeren der Formel I verwendet werden, keine H-aktiven Gruppen auf, jedenfalls keine ungeschützten H-aktiven Gruppen. Nachstehend werden diese als Gruppe-I-Vinylmonomere bezeichnet. Demgegenüber weisen diejenigen Vinylmonomere, die zur Herstellung von polymerisierbaren Verbindungen der Formel C verwendet werden, ausdrücklich mindestens eine H-aktive Gruppe auf. Nachstehend werden diese als Gruppe-C-Vinylmonomere bezeichnet.

Aryl bedeutet insbesondere einen aromatischen Kohlenwasserstoffrest mit 6 bis 15 Kohlenstoffatomen, wie Phenyl oder Phenyl, das durch einen oder mehrere, insbesondere bis zu drei, Reste der Art Niederalkyl, Niederalkoxy, Halogen, Amino oder Hydroxy substituiert ist. Beispiele sind Phenyl oder Tolyl.

Halogen bedeutet insbesondere Chlor, Brom oder Fluor, kann jedoch auch für Iod stehen.

Ein heterocyclischer Rest ist insbesondere ein 5- oder 6-gliedriger aromatischer oder gesättigter Ring mit ein oder zwei Heteroatomen, wie Sauerstoff- oder Stickstoffatomen, insbesondere mit ein oder zwei Stickstoffatomen. Hiervon sind auch Lactame erfasst.

Eine Kohlenwasserstoffbrücke, die ununterbrochen oder durch ein oder zwei Heteroatome unterbrochen ist, bedeutet insbesondere Niederalkylen oder durch Sauerstoff oder Stickstoff unterbrochenes Niederalkylen. Durch Stickstoff unterbrochenes Niederalkylen kann auch substituiert sein, z.B. durch Niederalkyl. Beispiele sind 1,3-Propylen, 2-Aza-1,3-Propylen oder N-Methyl-2-Aza-1,3-Propylen.

Acyl steht für Carboxy, Aroyl, Cycloalkanoyl oder Alkanoyl und insbesondere für Carboxy, unsubstituiertes oder substituiertes Aryloxycarbonyl, unsubstituiertes oder substituiertes Cycloalkyloxycarbonyl oder unsubstituiertes oder substituiertes Alkoxycarbonyl.

Aroyl bedeutet beispielsweise Benzoyl oder durch einen oder mehrere, insbesondere bis zu drei, Reste der Art Niederalkyl, Niederalkoxy, Halogen oder Hydroxy substituiertes Benzoyl, kann aber auch Phenylsulfonyl oder Phenyloxysulfonyl sowie durch Niederalkyl, Niederalkoxy, Halogen oder Hydroxy substituiertes Phenylsulfonyl oder Phenyloxysulfonyl bedeuten.

Alkanoyl bedeutet vorzugsweise Niederalkanoyl und ist z.B. Acetyl, Propanoyl oder Butanoyl.

Cycloalkanoyl bedeutet vorzugweise Cycloalkyloxycarbonyl mit bis zu 8 Kohlenstoffatomen und bedeutet z.B. Cyclohexyloxycarbonyl.

Unsubstituiertes Alkoxycarbonyl ist vorzugsweise Niederalkoxycarbonyl und bedeutet z.B. Methoxycarbonyl, Ethoxycarbonyl, Propyloxycarbonyl, Butoxycarbonyl, tert.-Butoxycarbonyl, tert-Butylmethyloxycarbonyl oder 2-Ethylhexyloxycarbonyl.

Unsubstituiertes Aryloxycarbonyl ist vorzugsweise Phenyloxycarbonyl.

Substituiertes Aryloxycarbonyl ist vorzugsweise durch einen oder mehrere, insbesondere bis zu drei, Reste der Art Niederalkyl, Niederalkoxy, Halogen oder Hydroxy substituiertes Phenyloxycarbonyl.

Substituiertes Alkoxycarbonyl ist vorzugsweise durch hydrophobe Gruppen, wie Halogen, z.B. Fluor, Siloxangruppen oder hydrophile Gruppen, wie Hydroxy, Amino, Mono- oder Diniederalkylamino, Isocyanato oder durch ein Niederalkylenglycol substituiert. Weitere Bedeutungen von substituiertem Alkoxycarbonyl, wie auch von substituiertem Aryloxycarbonyl und substituiertem Cycloalkyloxycarbonyl, werden implizit durch die nachfolgende Beschreibung von speziell geeigneten Vinylmonomeren der Formel III gegeben.

Die erfindungsgemäss verwendbaren hydrophilen Vinylmonomere sind vorzugsweise Acrylate und Methacrylate der Formel III, worin W und $Y_o$ für Wasserstoff stehen, $X_o$ für Wasserstoff oder Methyl steht und Z eine Gruppe $-Z^1-Z^2$ bedeutet, worin $Z^1$ für -COO-steht, das über Sauerstoff an $Z^2$ gebunden ist, und $Z^2$ einen durch eine wasserlöslich machende Gruppe wie Carboxy, Hydroxy oder tert.-Amino, z.B. tert. Niederalkylamino mit 1 bis 7 Kohlenstoffatomen je Niederalkylgruppe, eine Polyethylenoxidgruppe mit 2-100 sich wiederholenden Einheiten, bevorzugt mit 2-40 sich wiederholenden Einheiten, oder eine Sulfat-, Phosphat-, Sulfonat- oder Phosphonatgruppe einfach oder mehrfach substituierten Kohlenwasserstoffrest mit 1 bis 10 Kohlenstoffatomen bedeutet, wie z.B. einen entsprechend substituierten Alkyl-, Cycloalkyl- oder Phenylrest oder eine Kombination solcher Reste, wie Phenylalkyl oder Alkylcycloalkyl;
ferner Acrylamide und Methacrylamide der Formel III, worin W und $Y_o$ für Wasserstoff stehen, $X_o$ für Wasserstoff oder Methyl steht und Z Aminocarbonyl oder Diniederalkylaminocarbonyl bedeutet;
Acrylamide und Methacrylamide der Formel III, worin W und $Y_o$ für Wasserstoff stehen, $X_o$ für Wasserstoff oder Methyl steht und Z monosubstituiertes Aminocarbonyl bedeutet, das durch eine wie vorstehend definierte Gruppe $Z^2$ oder Niederalkyl substituiert ist;
Maleinate und Fumarate der Formel III, worin W und $X_o$ (oder W und Z) für Wasserstoff stehen, und $Y_o$ und Z (oder $X_o$ und $Y_o$) unabhängig voneinander eine Gruppe $-Z^1-Z^2$ bedeuten, worin $Z^1$ und $Z^2$ wie vorstehend definiert sind;
Crotonate der Formel III, worin W und $X_o$ für Wasserstoff stehen, $Y_o$ für Methyl steht und Z eine Gruppe $-Z^1-Z^2$ bedeutet, worin $Z^1$ und $Z^2$ wie vorstehend definiert sind;
Vinylether der Formel III, worin W, $X_o$ und $Y_o$ für Wasserstoff stehen und Z eine Gruppe $-Z^1-Z^2$ bedeutet, worin $Z^1$ für Sauerstoff steht und $Z^2$ wie vorstehend definiert ist;
Vinyl-substituierte fünf- oder sechsgliedrige Heterocyclen mit ein oder zwei Stickstoffatomen sowie N-Vinyllactame, wie N-Vinyl-2-pyrrolidon, der Formel III, worin W, $X_o$ und $Y_o$ für Wasserstoff stehen und Z einen fünf- oder sechsgliedrigen heterocyclischen Rest mit ein oder zwei Stickstoffatomen bedeutet, sowie den über Stickstoff gebundenen Rest eines Lactams, z.B. denjenigen von 2-Pyrrolidon;
und vinylisch ungesättigte Carbonsäuren der Formel III mit insgesamt 3 bis 10 Kohlenstoffatomen, wie Methacrylsäure, Crotonsäure, Fumarsäure oder Zimtsäure.

Bevorzugt sind z.B. durch Hydroxy oder Amino substituierte $C_2$-$C_4$-Alkyl(meth)acrylate, fünf- bis siebengliedrige N-Vinyllactame, N,N-Di-$C_1$-$C_4$-alkyl(meth)acrylamide und vinylisch ungesättigte Carbonsäuren mit insgesamt 3 bis 5 Kohlenstoffatomen. Unter diesen sind fünf- bis siebengliedrige N-Vinyllactame und N,N-Di-$C_1$-$C_4$-alkyl(meth)acrylamide Gruppe-I-Vinylmonomere, während durch Hydroxy oder Amino substituierte $C_2$-$C_4$-Alkyl(meth)acrylate und vinylisch ungesättigte Carbonsäuren mit insgesamt 3 bis 5 Kohlenstoffatomen Gruppe-C-Vinylmonomere sind. Die Unterscheidung der vor- und nachstehend genannten Vinylmonomere in Vertreter des Gruppe-I-Typs und des Gruppe-C-Typs wird hier nicht für alle Vertreter im einzelnen vorgenommen, da die Unterscheidung anhand des Kriteriums, ob eine H-aktive Gruppe vorliegt oder nicht, leicht zu treffen ist. Die beiden Gruppen von Vinylmonomeren werden aufgrund dieses Unterscheidungskriteriums als unabhängig voneinander offenbart angesehen.

Zu den verwendbaren wasserlöslichen Monomeren gehören: 2-Hydroxyethyl-, 2- und 3-Hydroxypropyl-, 2,3-Dihydroxypropyl-, Polyethoxyethyl- und Polyethoxypropylacrylate und -methacrylate sowie die entsprechenden Acrylamide und Methacrylamide, Acrylamid und Methacrylamid, N-Methylacrylamid und -methacrylamid, Bisaceton-acrylamid, 2-Hydroxyethylacrylamid, Dimethylacrylamid und -methacrylamid sowie Methylolacrylamid und -methacrylamid, N,N-Dimethyl- und N,N-Diethylaminoethylacrylat und -methacrylat sowie die entsprechenden Acrylamide und Methacrylamide, N-tert.-Butylaminoethylmethacrylat und -methacrylamid, 2- und 4-Vinylpyridin, 4- und 2-Methyl-5-vinylpyridin, N-Methyl-4-vinylpiperidin, 1-Vinyl- und 2-Methyl-1-vinyl-imidazol, Dimethylallylamin und Methyldiallylamin sowie para-, meta- und ortho-Aminostyrol, Dimethylaminoethylvinylether, N-Vinylpyrrolidon und 2-Pyrrolidinoethylmethacrylat, Acryl- und Methacrylsäure, Itaconsäure, Zimtsäure, Crotonsäure, Fumarsäure, Maleinsäure und deren Hydroxyniederalkylmono- und -diester, wie 2-Hydroxymethyl- und Di(2-hydroxy)-ethylfumarat, -maleinat und -itaconat, sowie 3-Hydroxypropyl-butylfumarat und Di-polyalkoxyalkyl-fumarate, -maleinate und -itaconate, Maleinsäureanhydrid,

N-Methyl-maleinsäureimid, Natriumacrylat und -methacrylat, 2-Methacryloyloxyethylsulfonsäure, 2-Acrylamido-2-methylpropansulfonsäure, 2-Phosphatoethylmethacrylat, Vinylsulfonsäure, Phenylvinylsulfonat, Natriumvinylsulfonat, p-Styrolsulfonsäure, Natrium-p-styrolsulfonat und Allylsulfonsäure, N-Vinylpyrrolidon, N-Vinylpyridon, N-Vinylcaprolactam, ferner die quaternisierten Derivate kationischer Monomerer, welche man durch Quaternierung mit ausgewählten Alkylierungsmitteln, z.B. halogenierten Kohlenwasserstoffen wie Methyliodid, Benzylchlorid oder Hexadecylchlorid, Epoxiden wie Glycidol, Epichlorhydrin oder Ethylenoxid, Acrylsäure, Dimethylsulfat, Methylsulfat und Propansulton erhält.

Eine vollständigere Liste im Zusammenhang mit dieser Erfindung verwendbarer, wasserlöslicher Monomerer findet sich in: R.H. Yocum und E.B. Nyquist, Functional Monomers [Funktionelle Monomere], Band 1, S. 424-440 (M. Dekker, N.Y. 1973).

Bevorzugte hydrophile Vinylmonomere sind 2-Hydroxyethylmethacrylat, 3-Hydroxypropylmethacrylat, N-Vinyl-2-pyrrolidon, Polyethylenglykolmethacrylat, insbesondere mit einem Ethylenglykolanteil eines Molekulargewichts von etwa 400, N,N-Dimethylacrylamid, N,N-Diethylaminoethyl(meth)acrylat sowie Acryl- und Methacrylsäure.

Als hydrophobe Vinylmonomere, die gegebenenfalls erfindungsgemäss verwendet werden, kommen beispielsweise in Betracht:

Acrylate und Methacrylate der Formel III, worin worin W und $Y_o$ für Wasserstoff stehen, $X_o$ für Wasserstoff oder Methyl steht und Z eine Gruppe $-Z^1-Z^3$ bedeutet, worin $Z^1$ für -COO- steht, das über Sauerstoff an $Z^3$ gebunden ist und $Z^3$ eine lineare oder verzweigte aliphatische, eine cycloaliphatische oder eine aromatische Gruppe mit 1 bis 21 Kohlenstoffatomen ist, wie z.B. ein entsprechend substituierter Alkyl-, Cycloalkyl oder Phenylrest oder eine Kombination solcher Reste, wie Phenylalkyl oder Alkylcycloalkyl, die Ether- oder Thioetherbindungen, Sulfoxid oder Sulfongruppen oder eine Carbonylgruppe enthalten kann; oder $Z^3$ eine heterocyclische Gruppe ist, die Sauerstoff-, Schwefel- oder Stickstoffatome und 5 oder 6, oder falls sie bicyclisch ist, bis zu 10 Ringatome enthält, oder eine Polypropylenoxid- oder Poly-n-butylenoxid-Gruppe mit 2 bis 50 wiederkehrenden Alkoxyeinheiten, oder $Z^3$ eine Alkylgruppe mit 1-12 Kohlenstoffatomen ist, die Halogenatome enthält, insbesondere Fluoratome, oder $Z^3$ eine Siloxangruppe mit 1 bis 6 Si-Atomen ist;

Acrylamide und Methacrylamide der Formel III, worin W und $Y_o$ für Wasserstoff stehen, $X_o$ für Wasserstoff oder Methyl steht und Z monosubstituiertes Aminocarbonyl bedeutet, das durch eine wie vorstehend definierte Gruppe $Z^3$ substituiert ist;

Maleinate und Fumarate der Formel III, worin W und $X_o$ (oder W und Z) für Wasserstoff stehen, und $Y_o$ und Z (oder $X_o$ und $Y_o$) unabhängig voneinander eine Gruppe $-Z^1-Z^3$ bedeuten, worin $Z^1$ und $Z^3$ wie vorstehend definiert sind;

Itaconate der Formel III, worin W und $Y_o$ für Wasserstoff stehen, $X_o$ eine Gruppe $-Z^1-Z^3$ bedeutet, worin $Z^1$ und $Z^3$ wie vorstehend definiert sind und Z eine Gruppe $-CH_2-Z^1-Z^3$ bedeutet, worin $Z^1$ und $Z^3$ wie vorstehend definiert sind;

Crotonate der Formel III, worin W und $X_o$ für Wasserstoff stehen, $Y_o$ für Methyl steht und Z eine Gruppe $-Z^1-Z^3$ bedeutet, worin $Z^1$ und $Z^3$ wie vorstehend definiert sind;

Vinylester der Formel III, worin W, $Y_o$, und $X_o$ für Wasserstoff stehen und Z eine Gruppe $-Z^1-Z^3$ bedeutet, worin $Z^1$ für -COO- steht, das über Kohlenstoff an $Z^3$ gebunden ist, und $Z^3$ wie vorstehend definiert ist;

Vinylether der Formel III, worin W, $X_o$ und $Y_o$ für Wasserstoff stehen und Z eine Gruppe $-Z^1-Z^3$ bedeutet, worin $Z^1$ für Sauerstoff steht und $Z^3$ wie vorstehend definiert ist;

Bevorzugt sind insbesondere $C_1$-$C_4$-Alkylester oder $C_5$-$C_7$-Cycloalkylester von vinylisch ungesättigten Carbonsäuren mit 3 bis 5 Kohlenstoffatomen.

Beispiele geeigneter hydrophober Monomerer sind: Methyl-, Ethyl-, Propyl-, Isopropyl-, Butyl-, Isobutyl-, tert-Butyl-, Ethoxyethyl-, Methoxyethyl-, Benzyl-, Phenyl-, Cyclohexyl-, Trimethylcyclohexyl-, Isobornyl-, Dicyclopentadienyl-, Norbornylmethyl-, Cyclododecyl-, 1,1,3,3-Tetramethylbutyl-, n-Butyl-, n-Octyl-, 2-Ethylhexyl-, Decyl-, Dodecyl-, Tridecyl-, Octadecyl-, Glycidyl-, Ethylthioethyl-, Furfuryl-, Tri-, Tetra- und Pentasiloxanylpropyl-acrylate und -methacrylate, sowie die entsprechenden Amide; N-(1,1-Dimethyl-3-oxobutyl)-acrylamid; Mono- und Dimethyl-fumarat, -maleat und -itaconat; Diethylfumarat; Isopropyl- und Diisopropyl-fumarat und -itaconat: Mono- und Diphenyl- und Methylphenyl-fumarat und -itaconat; Methyl- und Ethylcrotonat; Methylvinylether und Methoxyethylvinylether; Vinylacetat, Vinylpropionat, Vinylbenzoat, Acrylnitril, Vinylidenchlorid, Styrol, $\alpha$-Methylstyrol und tert-Butylstyrol.

Bevorzugte hydrophobe Vinylmonomere sind Methylmethacrylat n-Butylmethacrylat, Isopropylmethacrylat, Isobutylmethacrylat, Cyclohexylmethacrylat oder ein Gemisch davon.

Unter den vorstehend genannten Vinylmonomeren sind zwei spezielle Typen von hydrophoben Vinylmonomeren im erfindungsgemässen Zusammenhang besonders erwähnenswert, nämlich Siloxanmonovinylkomponenten und fluorhaltige Vinylverbindungen.

Besonders bevorzugte Siloxanmonovinylkomponenten sind Verbindungen der Formel III, worin W und $Y_o$ für Wasserstoff stehen, $X_o$ für Wasserstoff oder Methyl steht und Z eine Gruppe $-Z^1-Z^4$ bedeutet, worin $Z^1$ für -COO- steht, das über Sauerstoff an $Z^4$ gebunden ist und worin $Z^4$ ein oder mehrfach, z.B. drei- bis neunfach, durch Triniederalkylsilyloxy substituiertes Silyl-niederalkyl ist. Unter Silyl-niederalkyl wird in diesem Zusammenhang ein durch ein oder mehrere Siliciumatome substituierter Niederalkylrest verstanden, dessen freie Valenzen an den Siliciumatomen

insbesondere durch Triniederalkylsilyloxy abgesättigt sind. Speziell hervorzuhebende Einzelverbindungen sind beispielsweise Tris(trimethylsiloxy)silylpropylmethacrylat und Tris(tris(trimethylsiloxy)siloxy)silylpropylmethacrylat.

Besonders bevorzugte fluorhaltige Vinylverbindungen sind Verbindungen der Formel III, worin W und $Y_o$ für Wasserstoff stehen, $X_o$ für Wasserstoff oder Methyl steht und Z eine Gruppe $-Z^1-Z^5$ bedeutet, worin $Z^1$ für -COO- steht, das über Sauerstoff an $Z^5$ gebunden ist und worin $Z^5$ durch Fluor substituiertes Alkyl, insbesondere Niederalkyl ist. Spezielle Beispiele hierfür sind 2,2,2-Trifluorethylmethacrylat, 2,2,3,3-Tetrafluorpropylmethacrylat, 2,2,3,3,4,4,5,5-Octafluorpentylmethacrylat und Hexafluorisopropylmethacrylat.

Wie bereits erwähnt, werden die erfindungsgemässen Polymere vorzugweise ausgehend von einer Verbindung der Formel C oder D, gegebenfalls einem Vinylmonomer, und in Gegenwart von einem Vernetzer hergestellt.

Geeignete vinylische Vernetzer sind insbesondere oligoolefinische, insbesondere diolefinische Monomere, z.B. Allylacrylat und -methacrylat, Ethylenglykol-, Diethylenglykol-, Triethylenglykol-, Tetraethylenglykol- und allgemein Polyethylenoxidglykoldiacrylate und -dimethacrylate 1,4-Butandiol- und Polyn-butylenoxidglykoldiacrylate und -dimethacrylate, Propylenglykol- und Polypropylenoxidglykoldiacrylate und -dimethacrylate, Thiodiethylenglykoldiacrylat und -dimethacrylat, Di-(2-hydroxyethyl)-sulfondiacrylat und -dimethacrylat, Neopentylglykoldiacrylat und -dimethacrylat, Trimethylolpropan-tri- und -tetraacrylat, Pentaerythrit-tri- und -tetra-acrylat, Divinylbenzol, Divinylether, Divinylsulfon, Disiloxanyl-bis-3-hydroxypropyldiacrylat oder-methacrylat und verwandte Verbindungen. Ethylenglykoldimethacrylat ist bevorzugt.

Als Vernetzer kommen auch Oligovinylmacromere, z.B. Divinylmacromere in Frage, wie sie z.B. in der US-A-4,136,250 beschrieben sind. Ferner sind als Vernetzer im erfindungsgemässen Zusammenhang auch Oligovinylsiloxanverbindungen geeignet, z.B. Bis(meth)-acryloxy-niederalkyl-siloxane mit bis zu 10 Siliciumatomen. Beispiele hierfür sind 3,5-Bis(3-methacroyloxypropyl)-3,5-bis(trimethylsiloxy)-1,1,1,7,7,7-hexamethyltetrasiloxan und 1,3-Dimethacryloxypropyl-tetramethyldisiloxan.

Die bei der Herstellung der erfindungsgemässen Vinyltelomere, segmentierten Copolymere, polymerisierbaren Verbindungen und Polymere zur Anwendung kommenden Ausgangsmaterialien, z.B. solche der Formeln A, B, III und die Vernetzer sind an sich bekannt und / oder hierin beschrieben.

Die Verbindungen der Formeln II können in an sich bekannter Weise durch die Umsetzung von Diisocyanaten mit den entsprechenden H-aciden Photoinitiatoren hergestellt werden. Die Verbindungen werden in hohen Ausbeuten und Reinheiten erhalten, selbst wenn im Photoinitiator gleichzeitig zwei verschieden reaktive H-acide Gruppen zugegen sind, zum Beispiel zwei OH-Gruppen. Besonders vorteilhaft ist es, Diisocyanate mit Isocyanatgruppen unterschiedlicher Reaktivität zu verwenden, weil hiermit die Bildung von Isomeren und Diaddukten weitgehend unterdrückt werden kann. Die unterschiedliche Reaktivität kann zum Beispiel wie zuvor beschrieben durch eine sterischen Hinderung bewirkt werden. Die unterschiedliche Reaktivität kann auch durch eine Verkappung einer Isocyanatgruppe im Diisocyanat erzielt werden, zum Beispiel mit Carbonsäuren oder Hydroxylamin. Die Verbindungen der Formel IIa sind aus der EP-A-632329 bekannt.

Verbindungen der Formel (IIb) lassen sich dadurch herstellen, dass man eine Verbindung der Formel IIc

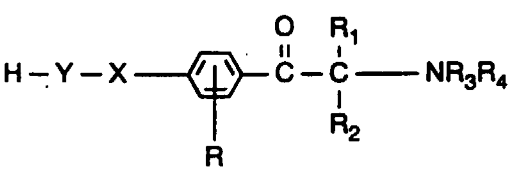

(IIc),

worin X, Y, R, $R_1$, $R_2$, $R_3$ und $R_4$ die zuvor angegebenen Bedeutungen haben, vorzugsweise in einem inerten organischen Lösungsmittel mit einem Diisocyanat der Formel IId oder einem solchen gegebenenfalls monoverkappten Diisocyanat,

$$OCN-R_5-NCO \quad \text{(IId)},$$

worin $R_5$ die zuvor angegebenen Bedeutungen hat, umsetzt.

Verkappungsmittel sind aus der Urethanchemie bekannt. Es kann sich zum Beispiel um Phenole (Kresol, Xylenol), Lactame (ε-Caprolactam), Oxime (Acetoxim, Benzophenonoxim), H-aktive Methylenverbindungen (Diethylmalonat, Ethylacetoacetat), Pyrazole oder Benztriazole handeln. Verkappungsmittel sind zum Beispiel von Z. W. Wicks, Jr. in Progress in Organic Coatings, 9(1981), Seiten 3-28 beschrieben.

Die Edukte vom Typ der Formel IIc sind bekannt und werden z.B. in EP-A-284,561, EP-A-117,233 oder EP-A-088,050 beschrieben.

Geeignete inerte Lösungsmittel sind aprotische unpolare oder polare Lösungsmittel wie zum Beispiel Kohlenwasserstoffe (Petrolether, Methylcyclohexan, Benzol, Toluol, Xylol), Halogenkohlenwasserstoffe (Chloroform, Methylenchiorid, Trichlorethan, Tetrachlorethan, Chlorbenzol), Ether (Diethylether, Dibutylether, Ethylenglykoldimethylether, Diethylenglykoldimethylether, Tetrahydrofüran (THF), Dioxan), Ketone (Aceton, Dibutylketon, Methyl-isobutylketon), Carbonsäureester und Lactone (Essigsäureethylester, Butyrolacton, Valerolacton), alkylierte Carbonsäureamide (N,N-Dimethylacetamid (DMA) oder N,N-Dimethylformamid (DMF), N-Methyl-2-pyrrolidon (NMP)), Nitrile (Acetonitril), Sulfone und Sulfoxide (Dimethylsulfoxid (DMSO), Tetramethylensulfon). Bevorzugt werden polare Lösungsmittel verwendet.

Die Reaktanden werden vorteilhaft in äquimolaren Mengen eingesetzt. Die Reaktionstemperatur kann zum Beispiel von 0 bis 200°C betragen. Bei der Verwendung von Katalysatoren können die Temperaturen zweckmässig im Bereich von -20° bis 60°C und vorzugsweise im Bereich von -10° bis 50°C liegen. Geeignete Katalysatoren sind mm Beispiel Metallsalze wie Alkalimetallsalze von Carbonsäuren, tertiäre Amine, zum Beispiel $(C_1$-$C_6$-Alkyl$)_3N$ (Triethylamin, Tri-n-butylamin), N-Methylpyrrolidin, N-Methylmorpholin, N,N-Dimethylpiperidin, Pyridin und 1,4-Diaza-bicyclooctan. Als besonders effektiv haben sich Zinnverbindungen erwiesen, besonders Alkylzinnsalze von Carbonsäuren, wie zum Beispiel Dibutylzinndilaurat, oder z.B. Zinndioctoat.

Sofern in Verbindungen der Formel IIc freie NH-Gruppen vorhanden sind, so können diese während der Reaktion mit einem Diisocyanat mit geeigneten Schutzgruppen zuerst geschützt und nachher durch Abspaltung der Schutzgruppen wieder freigesetzt werden. Geeignete Schutzgruppen sind dem Fachmann bekannt. Repräsentative Beispiele können beispielsweise aus T.W. Greene, "Protective Groups in Organic Synthesis", Wiley Interscience, 1981, entnommen werden.

Die Isolierung und Reinigung der hergestellten Verbindungen erfolgt nach bekannten Verfahren wie zum Beispiel Extraktion, Kristallisation, Umkristallisation oder chromatographischen Reinigungsmethoden. Die Verbindungen werden in hohen Ausbeuten und Reinheiten erhalten. Die Ausbeuten bei nicht optimierten Verfahren können über 85 % der Theorie betragen.

Die Umsetzung eines copolymerisierbaren Vinylmononomeren, das als Bestandteil "A" in das Vinyltelomer eingebaut wird, mit einem Photoinitiator der Formel B kann auf an sich bekannte Weise erfolgen. So kann ein Vinylmonomer, das als Bestandteil "A" in das Vinyltelomer eingebaut wird, bei Raumtemperatur oder bei einer Temperatur bis maximal zur Siedetemperatur des gegebenenfalls verwendeten Lösungsmittels in Abwesenheit oder Gegenwart eines geeigneten Lösungsmittels polymerisiert werden. Ein geeignetes Lösungsmittel zeichnet sich in diesem Zusammenhang dadurch aus, dass es keine H-aktiven Wasserstoffatome enthält, die mit der Isocyanatgruppe reagieren können, und dass es kein UV-Licht absorbiert. Beispiele hierfür sind beispielsweise ein Kohlenwasserstoff, insbesondere ein Cycloaliphat, wie Hexan, Methylcyclohexan, Benzol oder Toluol, ein Keton, wie Aceton, Methylisopropylketon oder Cyclohexanon, ein Ester wie Essigester, ein fluoriertes Lösungsmittel wie Hexafluoraceton, ein vorzugsweise cyclischer Ether, wie Diethylether, Dimethoxyethan, Dioxan oder Tetrahydrofuran, oder ein Amid, wie N-Methylpyrrolidon oder DMA, oder Dimethylsulfoxid oder Acetonitril, oder ein Gemisch von mehreren dieser Lösungsmittel. Die Reinigung erfolgt auf an sich bekannte Weise. Für die Vernetzungsreaktion zu erfindungsgemässen Polymeren können grundsätzlich gleiche Bedingungen angewandt werden.

Es ist vorteilhaft, die Umsetzung so zu steuern, dass die gebildeten Vinyltelomere ausfallen und durch Umlauffiltration aus dem Reaktionsgemisch laufend entfernt werden können. Darüber hinaus ist es vorteilhaft, wenn Lösungsmittel verwendet werden, die eine sensibilisierende bzw. beschleunigende Wirkung auf die Phototelomerisation ausüben.

Eine vorteilhafte Reaktionsführung kann darin bestehen, dass man nur bis zu einem Verbrauch von etwa 40% an Vinylmonomer umsetzt, um Nebenprodukte zu vermeiden. Dies gilt insbesondere, wenn alle Komponenten sich von Anfang an im Reaktionsgemisch befinden, da dann relativ schnell eine Verarmung an Photoinitiator auftreten kann, wodurch die Möglichkeit der Bildung von nicht-NCO-terminiertem Homopolymer begünstigt wird.

Alternativ hierzu kann es vorteilhaft sein, sowohl eine Lösung des Monomeren als auch eine Lösung des Photoinitiators während der UV-Bestrahlung simultan zuzudosieren.

Die Umsetzung eines Vinyltelomeren der Formel I mit einem Vinylmonomeren zur Herstellung einer Verbindung der Formel C kann einfach und auf in der Urethanchemie an sich bekannte Weise erfolgen. Dies gilt ebenso für die Umsetzung eines Vinyltelomeren der Formel I mit einem Makromer der Formel A.

Die Herstellung der erfindungsgemässen Polymeren kann auf an sich bekannte Weise erfolgen, z.B. unter den vorstehend bereits angegebenen Bedingungen, wobei es in diesem Verfahrensschritt nicht erforderlich ist, dass Lösungsmittel frei von H-aktiven Gruppen sind.

Geeignete Olefine der genannten Pfropfpolymerisation sind zum Beispiel Acrylamid, N,N-Dimethylacrylamid, Methacrylamid, Hydroxyethylmethacrylat, Glycerylmethacrylat, Oligoethylenoxidmono- und -bisacrylate, Ethylenglykoldimethacrylat, Methylenbisacrylamid, Vinylcaprolactam, Acrylsäure, Methacrylsäure, Fumarsäuremonovinylester, Vinyltrifluoracetat und Vinylencarbonat, wobei reaktive Ester anschliessend gegebenenfalls hydrolysiert werden können.

Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Thioxanthon-, Anthrachinon- und 3-Acylcumarinderivate sowie 3-(Aroylmethylen)-thiazoline.

Die Wirksamkeit der Photoinitiatoren lässt sich steigern durch Zusatz von Titanocenderivaten mit fluororganischen Resten, wie sie in den EP-A-122,223 und EP-A-186,626 beschrieben sind, z.B. in einer Menge von 1-20 %. Beispiele für solche Titanocene sind Bis(methylcyclopentadienyl)-bis(2,3,6-trifluorphenyl)-titan, Bis(cyclopentadienyl)-bis-(4-dibutylamin-2,3,5,6-tetrafluorphenyl)-titan, Bis(methylcyclopentadienyl)2-(trifluormethyl)phenyl-titan-isocyanat, Bis(cyclopentadienyl)-2-(trifluormethyl)phenyltitan-trifluoracetat oder Bis(methylcyclopentadienyl)-bis(4-decyloxy-2,3,5,6-tetrafluorphenyl)-titan. Für diese Gemische eignen sich vor allem flüssige $\alpha$-Aminoketone.

Insbesondere aus den erfindungsgemässen Polymeren können auf an sich bekannte Weise Formkörper, insbesondere Kontaktlinsen hergestellt werden. Dazu werden z.B. die erfindungsgemässen Polymere in zylindrischer Form polymerisiert, und die erhältlichen Stäbe nach Entformung in Scheiben oder Knöpfe zerteilt, die weiter mechanisch bearbeitet werden können, insbesondere durch Drehverfahren. Darüberhinaus können die erfindungsgemässen Formkörper respektive Linsen auch nach anderen an sich bekannten Verfahren wie Giessen in statischen Formen, Rotationsgiessen, Verpressen, Tiefziehen, Warmformen, Drehen oder Laserbearbeitung hergestellt werden. Diese Verfahrensschritte sind an sich bekannt und bedürfen daher für den Fachmann keiner detaillierten Erläuterung.

Die Herstellung erfolgt vorzugsweise unter einer inerten Atmosphäre, wenn sie in offenen Formen durchgeführt wird. Bekanntlich hemmt Sauerstoff die Polymerisation und führt zu verlängerten Polymerisationszeiten. Werden geschlossene Formen zur Bildung des Polymerisats verwendet, so bestehen die Formen vorteilhafterweise aus inerten Materialien mit niedriger Sauerstoffdurchlässigkeit und mit nicht-klebenden Eigenschaften. Beispiele für geeignete Formmaterialien sind Polytetrafluorethylen, wie Teflon®, Silikonkautschuk, Polyethylen, Polypropylen und Polyester wie Mylar®. Bei Einsatz eines geeigneten Entformungsmittels sind auch Formen aus Glas und Metall verwendbar.

Giessen in statischen Formen kann beispielsweise, wenn Formen mit Innenkurve und Aussenkurve verwendet werden, unmittelbar zu Kontaktlinsen führen. So können Kontaktlinsen durch Polymerisation in geeigneten Formen direkt ("full mold"-Verfahren) oder mit nur einer fertigen Fläche ("semi mold"-Verfahren) hergestellt werden.

Rotationsgiessen (spin casting) lässt sich erfindungsgemäss ebenfalls anwenden, indem eine Lösung der erfindungsgemässen Ausgangsmaterialien in eine Form für Rotationsguss eingebracht wird, worauf die Form in Rotation versetzt wird. Dabei verdampft das Lösungsmittel. Die fertige Kontaktlinse, deren Abmessungen sich durch die Abmessungen der Form, die Rotationsgeschwindigkeit und die Viskosität der eingebrachten Lösung steuern lassen, bleibt in der Form zurück.

Verpressen geschieht erfindungsgemäss z.B. durch Formpressen einer Folie aus dem erfindungsgemässen Polymer. Eine Folie aus dem Polymer kann auf an sich bekannte Weise beispielsweise durch Giessen einer Lösung hergestellt werden.

Aus einer z.B. wie vorstehend erwähnt hergestellten Folie kann eine Kontaktlinse auch auf an sich bekannte Weise durch Tiefziehen oder Warmformen hergestellt werden.

Drehen bietet sich als letzter Verfahrensschritt zur Herstellung von erfindungsgemässen Kontaktlinsen ebenfalls an. Dies gilt immer dann, wenn ein z.B. nach einem der vorstehend genannten Verfahren erhältlicher Rohling noch weiterer Bearbeitung bedarf. Unter Drehen wird das an sich bekannte spanabhebende Bearbeitungsverfahren von Kontaktlinsen-Rohlingen verstanden. Entsprechende Rohlinge lassen sich z.B. durch Extrusion von Rundstäben und deren Zerteilen oder Giessen aus einer Lösung herstellen. Unter den Begriff Kontaktlinsen-Rohling fallen in diesem Zusammenhang Knöpfe (buttons) oder semi-mold-Produkte, wie z.B. Innenkurvenrohlinge. Typische Rohlinge weisen Dicken von 4 oder 6 mm und Durchmesser von 10 bis 17, z.B. 12 oder 14 mm auf. Für weiche Materialien kann es erforderlich sein, diese vor einer entsprechenden Bearbeitung zu gefrieren, insbesondere unter den Erweichungspunkt, und die hierfür erforderlichen Temperaturen nötigenfalls während der Bearbeitung aufrecht zu erhalten.

Auch die Laserbearbeitung lässt sich erfindungsgemäss anwenden, wobei man von Rohlingen oder nach einem der anderen Verfahren hergestellten Kontaktlinsen ausgeht, sofern letztere noch einer zusätzlichen Feinbearbeitung ihrer Oberfläche bedürfen.

Die Beschichtung einer Oberfläche mit einem Vinyltelomer der Formel I wird auf an sich bekannte Weise ausgeführt, wobei die Isocyanatgruppe des Vinyltelomeren mit H-aktiven Gruppen der Oberfläche reagiert. Dabei enthält die Oberfläche bereits entsprechende H-aktive Gruppen oder diese werden auf der Oberfläche auf an sich bekannte Weise zuvor erzeugt, wie beispielsweise durch Plasmabehandlung (verwiesen wird hierzu z.B. auf die WO 94/06485).

Das erfindungsgemässe Beschichtungsverfahren lässt sich durch die folgenden Schritte kennzeichnen:

a) die zu beschichtende Oberfläche wird, falls sie nicht bereits H-aktive Gruppen enthält, durch eine chemische oder physikalische Behandlung, z.B. eine Plasmabehandlung, mit H-aktiven Gruppen versehen, die mit Isocyanatgruppen coreaktiv sind, wobei es sich insbesondere um Gruppen $R_x$-H handelt, worin $R_x$ unabhängig voneinander für -O-, -$NR_N$- oder -S- steht, worin $R_N$ für Wasserstoff oder Niederalkyl steht,

b) die Oberfläche enthaltend H-aktive Gruppen, die mit Isocyanatgruppen coreaktiv sind, wird mit einem Vinyltelomer der Formel I, wie vorstehend beschrieben, in Kontakt gebracht, wobei die Isocyanatgruppen des Vinyltelomeren kovalente Bindungen mit den H-aktiven Gruppen der Oberfläche ausbilden.

Bei den Oberflächen handelt es sich vorzugsweise um die Oberflächen von Kontaktlinsen. Die Struktur der erhaltenen Oberflächenschichten entspricht einer sogenannten Bürstenstruktur, die äusserst vorteilhaft ist.

Die nachfolgenden Beispiele erläutern den Gegenstand der Erfindung, ohne ihn jedoch, etwa auf den Umfang der Beispiele, zu beschränken. Prozente bei Mengenangaben sind Gewichtsprozente soweit nicht ausdrücklich anders angegeben. In den nachfolgenden Beispielen sind Temperaturen, wenn nicht anders angegeben, in Grad Celsius offenbart, Molekulargewichte, wie auch sonst in dieser Beschreibung, sind mittlere Molekulargewichte (Bezeichnung: "Mw"), wenn nicht ausdrücklich anders angegeben. Die erfindungsgemässen Vinyltelomere werden in dieser Erfindungsbeschreibung auch als Phototelomere bezeichnet.

A-Beispiele: Herstellung von OCN-funktionellen Photoinitiatoren

<u>Beispiel A1:</u> Herstellung von

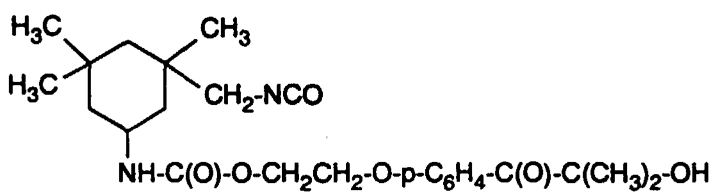

In einem 500 ml Kolben mit Rückflusskühler, Thermometer, Rührer und Stickstoffeinleitungsrohr wird unter Stickstoff eine Lösung von 11,125g (0,05 Mol) frisch destilliertem Isophorondiisocyanat (IPDI) in 50 ml trockenem Methylenchlorid mit einer Lösung von 11,2 g (0,05 Mol) 4'-(β-Hydroxyethoxy)-2-hydroxyprop-2-yl-phenon (Darocure® 2959) in 300 ml trockenem Methylenchlorid gemischt und nach Zugabe von 20 mg Dibutyl-zinndilaurat als Katalysator 48 Stunden bei Raumtemperatur gerührt. Der Verlauf der Umsetzung wird mittels Dünnschichtchromatographie auf Kieselgelplatten (60 $F_{254}$, Art. 5719 Merck) verfolgt (Laufmittel: Toluol/Acetonitril 7:3). Das entstandene Produkt wird durch Säulenchromatographie an Kieselgel 60 (Eluent Toluol/Acetonitril 7:3) von geringen Mengen an nicht umgesetztem Darocure® 2959 und zweifach substituiertem IPDI befreit. Nach Eindampfen der reinen Fraktionen am Rotationsverdampfer wird ein farbloses Oel erhalten, das beim Abkühlen auf -16°C langsam kristallisiert und anschliessend aus trockenem Diethylether umkristallisiert wird. Es werden 15,6g eines weissen kristallinen Produktes erhalten (70 % d. Th.), das einen Schmelzpunkt von 76°C aufweist.

Der Isocyanatgehalt des Produktes wird durch Titration mit Dibutylamin in Toluol ermittelt: berechnet 2,242 mVal/g, gefunden 2,25 mVal/g.

Die Methode ist in "Analytical Chemistry of Polyurethanes" (High Polymer Series XVI/Part III, D.S. David + H.B. Staley editors, Interscience Publishers, New York 1969 p. 86) beschrieben.

<u>Beispiel A2:</u> Herstellung von

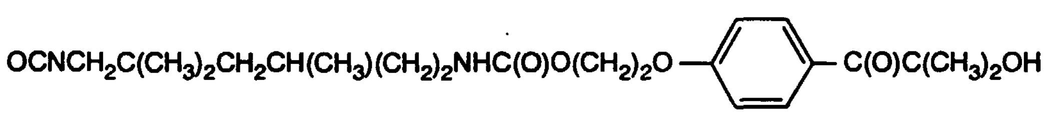

Analog Beispiel A1 werden 10,5 g (0,05 Mol) 1,6-Diisocyanato-2,2,4-trimethyl-hexan (TMDI) mit 11,1 g (0,05 Mol) Darocure® 2959 in 400 ml trockenem Methylenchlorid bei Raumtemperatur unter Stickstoff während 40 Stunden umgesetzt. Es werden 14,5g (67% d. Th.) eines weissen kristallinen Produktes vom Schmelzpunkt 41-43°C erhalten. NCO Titration: berechnet 2,30 mVal/g, gefunden 2,36 mVal/g.

Beispiel A3: Herstellung von

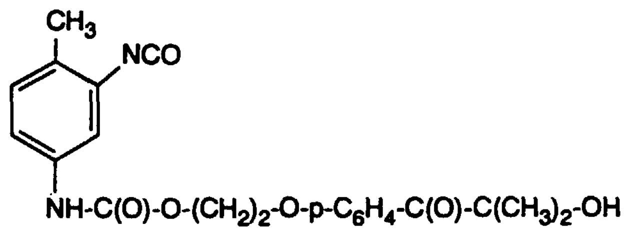

In der unter Beispiel A1 beschriebenen Apparatur werden 1,74 g (0,01 Mol) Toluylen-2,4-diisocyanat (TDI) in 20 ml Dichlormethan mit 2,24g (0,01 Mol) Darocure® 2959 gelöst in 60 ml trockenem Dichlormethan umgesetzt. Die Reaktionsmischung wird ohne Zusatz eines Katalysators während 48 Stunden bei Raumtemperatur und 1 Stunde bei 40°C gerührt, bis im Dünnschichtchromatogramm kein unumgesetztes Darocure® 2959 mehr nachgewiesen werden kann. Das Produkt wird durch Ausfällen der Reaktionslösung in 180 ml trockenem Petrolether (Sdp. 40-60°C) isoliert und anschliessend zweimal aus Dichlormethan/Petrolether 1:3 umkristallisiert.

Es wird ein weisses kristallines Produkt vom Schmelzpunkt 124-125°C erhalten. Ausbeute 17,2g entsprechend 87 % der Theorie. OCN-Titration: berechnet 2,50 mVal/g gefunden 2,39 mVal/g.

Beispiel A4:

Herstellung der nachstehenden Verbindung:

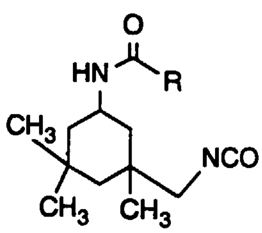

R=

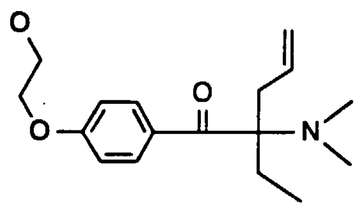

In einem 100 ml Kolben mit Rückflusskühler, Thermometer, Rührer und Stickstoffeinleitungsrohr werden 2.92g (10 mmol) 2-Ethyl-2-dimethylamino-1-(4-(2-hydroxyethoxy)-phenyl)-pent-4-en-1-on in 30 ml trockenem Methylenchlorid gelöst, und mit 2.22g (10 mmol) IPDI gelöst in 30 ml trockenem Methylenchlorid vermischt. Hierzu gibt man 2.0 mg des Katalysators DBTDL und rührt 72 Std. bei RT. Der Reaktionsverlauf wird mit DC verfolgt (Laufmittel ist Toluol / Aceton 6:1). Danach wird die Reaktionslösung in Wasser eingerührt. Die organische Phase wird abgetrennt und noch zweimal mit Wasser gewaschen. Die organische Phase wird über $MgSO_4$ getrocknet und am RV eingeengt. Der verbleibende Rückstand wird säulenchromatographisch gereinigt (Toluol / Aceton 6:1). Es verbleiben 3.4 g (66%) eines gelben Oeles. Die Struktur wird mit Protonen-NMR, IR und Elementaranalyse verifiziert.

Die Herstellung von 2-Ethyl-2-dimethylamino-1-(4-(2-hydroxyethoxy)phenyl)-pent-4-en-1-on

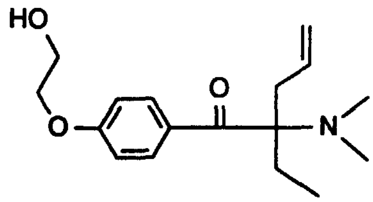

erfolgt in Anlehnung an die in EP-A-284,561 beschriebene Synthese in quantitativer Ausbeute. Es verbleiben gelbliche Kristalle vom Smp. 80 - 82°C.

Beispiel A5:

In Analogie zu Beispiel A4 wird das nachfolgende Isocyanat hergestellt aus 1.17 g (4 mmol) 1(4-(2-Hydroxyethoxy)phenyl)-2-methyl-2-morpholino-propan-1-on, 0.7g (4 mmol) 2,4-TDI mit DBTDL als Katalysator in Methylenchlorid. Nach der Zugabe von 50 ml Ether und 200 ml Petrolether zum Reaktionsgemisch fällt die Zielverbindung in kristalliner Form aus. Diese wird abfiltriert, mit Petrolether gewaschen und dann im Vakuum getrocknet. Man erhält die nachstehend wiedergegebene Verbindung mit einem Smp.von 97 - 102°C.

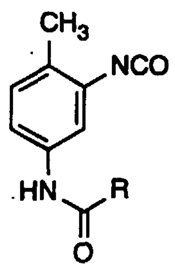

R=

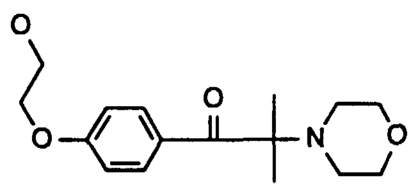

Die Herstellung von 1-(4-(2-Hydroxyethoxy)phenyl)-2-methyl-2-morpholino-propan1-on

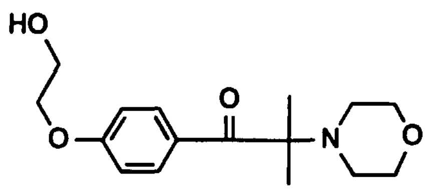

erfolgt in Anlehnung an die in der EP-A-088,050 beschriebene Synthese.

Beispiele A6:

In Analogie zu Beispiel A4 wird die nachstehende Verbindung hergestellt

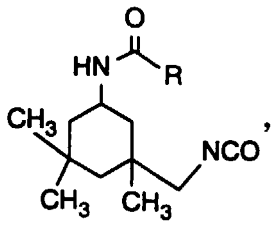 , wobei R für den Rest 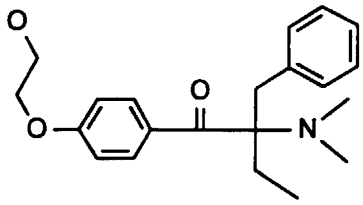 steht.

Beispiel A7:

In Analogie zu Beispiel A4 wird die nachstehende Verbindung hergestellt:

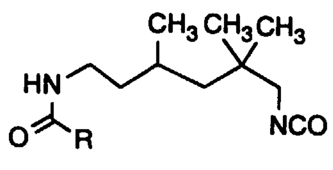

R= 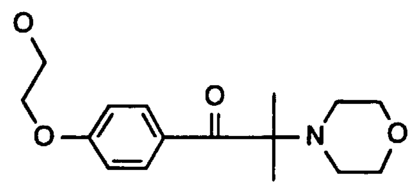

B-Beispiele:

Beispiel B1:

3 g (6,72 x $10^{-3}$ Mol) des in Beispiel A1 hergestellten Photoinitiators sowie 7,5 g (6,72 x $10^{-2}$ Mol) frisch destilliertes N-Vinylpyrrolidon werden in 60 ml trockenem Aceton gelöst und in einen DEMA-3H Photoreaktor eingefüllt, der eine wassergekühlte Hg-Hockdruck-Tauchlampe aufweist. Durch dreimaliges Evakuieren und Belüften mit trockenem Stickstoff wird die Lösung im Reaktor anschliessend von Sauerstoff befreit. Die so vorbereitete Lösung wird unter trockenem Stickstoff sowie unter starkem Rühren während 100 Minuten mit UV-Licht bestrahlt. In dieser Zeit setzt sich ein leicht gelbliches Festprodukt an der Wandung des Gefässes ab, das mit trockenem DMSO herausgelöst werden konnte, nachdem die Aceton-Reaktionslösung abgezogen worden war. Durch Ausfällen der beiden so erhaltenen Lösungen in dem 10-fachen Volumen an Nichtlösungsmittel (trocken!) werden zwei Feststoffe erhalten Produkt A und Produkt B.

**Produkt A:**
Lösung in 50 ml DMSO gefällt in 500 ml trockenem Diethylether,
Menge: 1,4g weisses Pulver, nach Trocknen im Hochvakuum über $P_2O_5$, 14 Stunden,
OCN-Gehalt: 0.041 mVal/g, durch Titration ermittelt,
IR-Spektrum: deutliche OCN-Absorptionsbande bei 2145 $cm^{-1}$,
Molekulargewicht: berechnet aus dem OCN-Gehalt: Mn etwa 25000 D, gemessen (Dampfdruckosmometrie) in DMF: Mn etwa 27600 +/- 3100 D,
Mittlerer Polymerisationsgrad: berechnet aus dem Molekulargewicht: DPn etwa 220.

**Produkt B:** Reaktionslösung in 50 ml Aceton ausgefüllt in 500 ml trockenem Diethylether,
Menge: 1,6 g weisses Pulver,
OCN-Gehalt: 0.191 mVal/g,
IR-Spektrum: starke OCN-Absorptionsbande bei 2145 $cm^{-1}$,

Molekulargewicht: berechnet aus dem OCN-Gehalt: Mn etwa 5260 D, gemessen (Dampfdruckosmometrie) in DMF: Mn etwa 5370 +/- 400 D,
Mittlerer Polymerisationsgrad: DPn etwa 44.

Beispiel B2:

Wie in Beispiel B1 beschrieben werden 3 g des funktionellen Photoinitiators und 7,5 g N-Vinylpyrrolidon unter analogen Reaktionsbedingungen miteinander umgesetzt. Es werden jedoch zunächst nur 30 ml Aceton im Photoreaktor vorgelegt. Dann werden unter ständiger UV-Bestrahlung die Lösung von 3 g Photoinitiator in 20 ml Aceton sowie die Lösung von 7,5 g NVP in 20 ml Aceton simultan über 5 Stunden langsam zugetropft. Nach einer weiteren halben Stunde Bestrahlungszeit wird das Reaktionsprodukt, das in diesem Fall ganz in Lösung geblieben war, durch Ausfällen in 700 ml trockenem Diethylether isoliert und getrocknet. Ausbeute 5,63 g (53,6 % der Theorie), OCN-Gehalt: 0,266 mVal/g, Molekulargewicht: Mn etwa 3760.

Beispiele B3 bis B10:

Analog Beispiel B2 wird eine Reihe weiterer hydrophiler und hydrophober Vinylmonomere zu OCN-funktionellen Phototelomeren umgesetzt:

Beispiel B3: 3 g Photoinitiator aus Beispiel A1,

| | | |
|---|---|---|
| Vinylmonomer: | 10 g DMA | |
| OCN-Gehalt (mVal/g): | 0,236 | Mn: 4240 |

Beispiel B4: 3 g Photoinitiator aus Beispiel A2,

| | | |
|---|---|---|
| Vinylmonomer: | 12 g Diethylenglykolmonomethylethermethacrylat | |
| OCN-Gehalt (mVal/g): | 0,122 | Mn: 8200 |

Beispiel B5: 3 g Photoinitiator aus Beispiel A3,

| | | |
|---|---|---|
| Vinylmonomer: | 12 g 2-(N,N-Dimethylaminoethyl)methacrylat | |
| OCN-Gehalt (mVal/g): | 0,568 | Mn: 1760 |

Beispiel B6: 3 g Photoinitiator aus Beispiel A4,

| | | |
|---|---|---|
| Vinylmonomer: | 17 g TRIS | |
| OCN-Gehalt (mVal/g): | 0,444 | Mn: 2250 |

Beispiel B7: 3 g Photoinitiator aus Beispiel A1,

| | | |
|---|---|---|
| Vinylmonomer: | 10 g MMA | |
| OCN-Gehalt (mVal/g): | 0,068 | Mn: 14700 |

Beispiel B8: 3 g Photoinitiator aus Beispiel A1,

| | | |
|---|---|---|
| Vinylmonomer: | 22 g 2,2,3,4,4,4-Hexafluorbutylmethacrylat | |
| OCN-Gehalt (mVal/g): | 0,30 | Mn: 3340 |

Beispiel B9: 3 g Photoinitiator aus Beispiel A1,

| | | |
|---|---|---|
| Vinylmonomer: | 20 g 3-(Pentamethyldisiloxy)propylmethacrylat | |
| OCN-Gehalt (mVal/g): | 0,140 | Mn: 7160 |

Beispiel B10: 3 g Photoinitiator aus Beispiel A1,

| | | |
|---|---|---|
| Vinylmonomer: | 15 g Glycidylmethacrylat | |
| OCN-Gehalt (mVal/g): | 0,089 | Mn: 11200 |

C-Beispiele: Oberflächenmodifizierung mittels OCN-Phototelomeren an Filmen und flachen Substraten

Beispiele C1-C4:

Filme aus verschiedenen Polymermaterialien, die reaktive Gruppen aufweisen, werden auf der Oberfläche mit der Lösung des gemäss Beispiel B1 hergestellten Phototelomeren in einem geeigneten Lösungsmittel (Konz. ~ 20 Gew.%) benetzt, wobei als Technik Tauchen, Sprühen und Bestreichen angewendet wird. Die so behandelten Filme werden unter trockenem Stickstoff 24 Stunden auf 60°C erhitzt und anschliessend durch Waschen mit Aceton von nicht umgesetztem Phototelomer befreit. Nach dem Trocknen unter Lichtausschluss werden die Filme mittels FTIR-Mikroskopie analysiert.

| Beispiel | Polymerfilm | $\overline{M}n$ | Lösungsmittel | IR-Banden ($cm^{-1}$) |
|---|---|---|---|---|
| C1 | Polyvinyl-alkohol | ~ 70'000 | DMSO | PVP: 1660 (C=O)<br>1440-1470 (C-H)<br>1280 (C-N) |
| C2 | Chitosan | ~ 145'000 | DMSO | PVP: 1660 (C=O)<br>1440-1470 (C-H)<br>1280 (C-N) |
| C3 | Collagen | ~ 80'000 | DMSO | PVP: 1660 (C=O)<br>1440-1470 (C-H)<br>1280 (C-N) |
| C4 | Polyvinyl-alkohol vernetzt mit 1 % TMDI | - | MEK+1 % DMSO | PVP: 1660 (C=O)<br>1440-1470 (C-H)<br>1280 (C-N) |
| TMDI = Trimethylhexandiisocyanat, MEK = Methylethylketon | | | | |

Beispiel C5:

Flache Platten (5 x 5 x 0,5 cm) aus a) Polyurethan, b) Glas und c) mit Kapton-Polyimid beschichtetem Aluminium werden einer herkömmlichen Behandlung mit einem Argon-Plasma in Gegenwart von n-Heptylamin unterworfen. Durch dieses Verfahren wird ein Film von einigen Nanometern Dicke auf den Substraten erzeugt, der reaktive Aminogruppen aufweist. Wie in den Beispielen C1 bis C4 beschrieben werden die so vorbehandelten Platten mit einer Lösung des Produktes A aus Beispiel B1 in DMSO behandelt. Auf diese Weise wird ein hydrophiles coating aus Poly-Vinylpyrrolidon auf den Platten erzeugt, das eine Bürstenstruktur aufweist. Folgende Kontaktwinkel werden mit einem Krüss G40 Instrument an diesen Platten gemessen:

| | Substrat a) | Substrat b) | Substrat c) |
|---|---|---|---|
| Kontaktwinkel unbehandelt [°]: | 78 | 46 | 96 |
| Kontaktwinkel behandelt [°]: | 38 | 36 | 42 |

Beispiel C6: (Plasma-behandelter Silikonfilm)

Ein Silikongummi-Film, der durch UV-Härtung von PS-2067 (Petrarch Hüls America Inc., Bristol USA), das auf einer Folanorm Folie (Folex, Zürich, Schweiz) zu einem Ueberzug aufgestrichen wurde, erzeugt wurde, wird bei 80°C und $10^{-3}$ Torr ausgeheizt. Anschliessend wird der Film in einen handelsüblichen 13,6 Megahertz Radiofrequenz Plasmareaktor plaziert, und das System wird auf 0,1 mbar evakuiert. Bei diesem Druck, einem Sauerstoff-Fluss von 10 Standardkubikzentimetern und 40 Watt Stromleistung wird der Film 30 Sekunden einem Sauerstoffplasma ausgesetzt. Nach Abschalten des Plasmas und Belüften des Reaktors wird der Film an der Luft gelagert.

Beispiel C7: (Polybutadienfilm, Plasma-behandelt)

Auf einer Folanorm Trägerfolie wird aus einer THF-Lösung von Poly-1,2-butadien (syndiotaktisches Polybutadien, Polysciences Inc, Produkt 16317) in einer Stickstoffatmosphäre ein 0,5 Millimeter dicker Film gegossen. Der Film wird wie in Beispiel C6 beschrieben einer Behandlung mit Sauerstoffplasma unterzogen.

Beispiel C8:

Aus einer Mischung von 92 % 2-Hydroxyethylmethacrylat (HEMA), 7,7 % Ethylenglykoldimethacrylat und 0,3 % Irgacure® 184 (0,3 %) wird auf einer Folanorm Trägerfolie ein Film gegossen und durch UV-Bestrahlung in üblicher Weise ausgehärtet.

Beispiel C9:

Eine 10 %-ige DMSO-Lösung von 99 % Polyvinylalkohol (PVA), Mn 72000, Fluka AG, Schweiz), und 1 % Isophorondiisocyanat (Aldrich) wird auf Folanorm zu einem Film ausgestrichen und durch zweistundiges Erhitzen auf 70°C zuletzt bei 0,01 Ton ausgehärtet. Der Film wird durch Waschen mit THF von DMSO sowie überschüssigem IPDI und anschliessend während 6 Stunden bei 80°C und 0,001 Ton von restlichem Lösungsmittel befreit.

Beispiele C10 bis C13:

Die folgende Tabelle zeigt die Kontaktwinkel (Krüss G40) von Polymerfilmen wie in den Beispielen C6 bis C9 beschrieben, die nach der in Beispiel C1 wiedergegebenen Methode mit dem in Beispiel B2 hergestellten OCN-funktionellen NVP-Phototelomer behandelt wurden.

| Beispiel | Material (Film) | Kontaktwinkel [°] | |
|---|---|---|---|
| | | unbehandelt | behandelt |
| C10 | Silikon (C6) | 100,4 | 54,5 |
| C11 | Polybutadien (C7) | 79,5 | 46,2 |
| C12 | Poly-HEMA (C8) | 78,4 | 38,5 |
| C13 | PVA (C9) | 47,1 | 32,5 |

Beispiel C14:

Von Salzen freigewaschene und gefriergetrocknete weiche Hydrogellinsen (STD-Cibasoft™, Tefilcon, CIBA Vision, Atlanta, USA) auf der Basis von vernetztem Poly-HEMA werden während 12 Stunden mit einer Lösung eines Phototelomeren (1 g in 10 ml trockenem DMSO), die 10 mg Dibutylzinndilaurat als Katalysator enthält, behandelt. Eingesetzt werden die Phototelomeren, die in den Beispielen B1 (A), B1 (B), B3, B4 und B5 beschrieben sind. Danach werden die Linsen sorgfältig mit Aceton und mit Wasser gewaschen und im Vakuum bei 0,1 mbar getrocknet. Die folgende Tabelle gibt die Kontaktwinkel der so behandelten und anschliessend in phosphatgepufferter (pH 7,4) physiologischer Kochsalzlösung autoklavierten (120°C, 30 Minuten) Linsen an. Die Daten zeigen, dass durch die Behandlung eine hydrophile Oberfläche auf den Linsen erzeugt wurde.

| Beispiel | Phototelomer von Beispiel | Kontaktwinkel | |
|---|---|---|---|
| | | unbehandelt | behandelt |
| a) | B1 (A) | 78 | 52 |
| b) | B1 (B) | 78 | 46 |
| c) | B3 | 78 | 42 |
| d) | B4 | 78 | 36 |
| e) | B5 | 78 | 50 |

Beispiel C15:

Verschiedene Kontaktlinsen werden in üblicher Weise einer Plasmabehandlung unterzogen, in Gegenwart von Ammoniakgas oder von n-Heptylamin, um reaktive Aminogruppen auf der Oberfläche zu erzeugen. die so vorbehandelten Linsen werden anschliessend wie in Beispiel C1 beschrieben mit einer Lösung des in Beispiel B2 hergestellten PVP-Phototelomeren behandelt. Die in der nachfolgenden Tabelle angegebenen Kontaktwinkel (Krüss G40 Instrument) zeigen, dass die behandelten und anschliessend autoklavierten Kontaktlinsen eine hydrophile Oberfläche besitzen.

| Beispiel | Kontaktlinsen-Material | Plasmagas | Kontaktwinkel | |
|---|---|---|---|---|
| | | | unbehandelt | behandelt |
| a) | Tefilcon | Ammoniak | - | 32 |
| b) | Silikon* | Ammoniak | 104 | 52 |
| c) | Silikon* | Heptylamin | 104 | 48 |
| d) | Atlafilcon A | Heptylamin | - | 37 |

*Das eingesetzte Silikonmaterial ist ein Copolymer, das aus 15 Gew.-% Methylmethacrylat, 15 Gew.-% TRIS und 70 Gew.-% eines Polydimethylsiloxan-Makromeren mit einem mittleren Molekulargewicht Mn von etwa 4000 besteht, das zwei terminale Hydroxybutylgruppen besitzt, die jeweils mit Isocyanatoethylmethacrylat terminiert wurden.

D-Beispiele:

Beispiel D1: Herstellung eines Kammpolymeren unter Verwendung eines OCN-funktionellen Phototelomeren

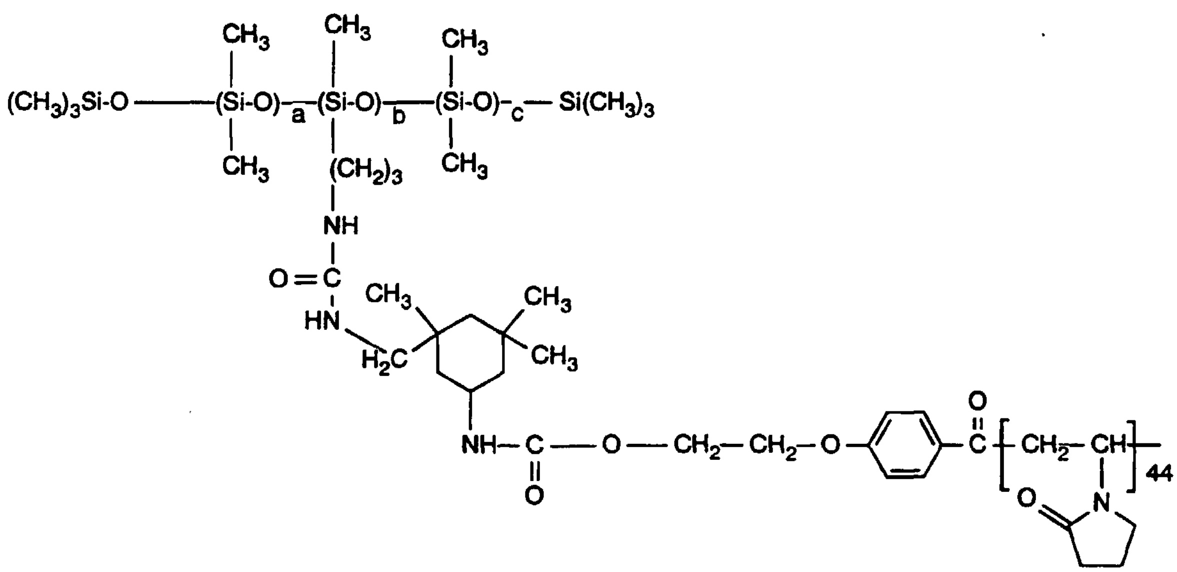

In einem 250 ml Kolben mit Rückflusskühler, Thermometer, Rührer und Stickstoffeinleitungsrohr wird unter trockenem Stickstoff eine Lösung von 11,78g (0,00224 Mol) des in Beispiel B1 beschriebenen OCN-terminierten Poly-(N-vinylpyrrolidon)-Telomeren B in 100 ml trockenem DMSO mit 4,37 g Aminoalkyl-polydimethylsiloxan (0,515 mVal $NH_2$/g, Petrarch PS 813®: $\overline{M}n \sim 3000$, b = 3, a+c = 37 ) gelöst in 50 ml trockenem Dichlormethan umgesetzt. Die Reaktionsmischung wird 2 Stunden bei Raumtemperatur gerührt und anschliessend 1 Stunde auf 40°C erwärmt. Nach dem Verdampfen des Dichlormethans am Rotationsverdampfer wird die viskose DMSO-Lösung des amphiphilen PDMS-Poly-NVP-Kammpolymeren durch Ausfällen in 1000 ml trockenem Diethylether isoliert. Nach dem Entfernen von Lösungsmittelresten zuletzt im Hochvakuum bei 40°C und $10^{-4}$ Torr werden 15,9g (98 % der Theorie) eines zähviskosen farblosen Produktes erhalten, das oberflächenaktive Eigenschaften aufweist. Das IR-Spektrum zeigt keine OCN-Absorption mehr.

Beispiele D2-D6:

Analog Beispiel D1 werden weitere Block- und Kammpolymere aus den beschriebenen OCN-funktionellen Phototelomeren durch Umsetzung mit aminofunktionellen Makromeren erhalten. Die Resultate sind in der nachfolgenden Tabelle zusammengefasst, wobei in den Strukturformeln "$Z_x$" den jeweiligen über eine Harnstoffbrücke gebundenen Rest des Phototelomeren bedeutet. Dabei gibt der Index "x" die jeweilige Nummer des Phototelomer-Beispiels aus der Reihe der B-Beispiele an.

Tabelle:

| Beispiel | aminofunktionelles Makromer | Verbindung nach Bsp. B1 - B10 | Struktur (Produkt) | Ausbeute |
|---|---|---|---|---|
| D2 | X-22-161c (Shin Etsu, JP) 7,8 g (0,43 mVal $NH_2$/g) $\overline{M} \sim 4600$ | 12,63 g B1A (3,36 mMol) | a | 19,6 g (96%) |
| D3 | Jeffamin® T 403 | 14,3 g B6 | b | 17,0g |

| | | | | |
|---|---|---|---|---|
| | (Texaco, USA)<br>2,8 g (6,38 mVal $NH_2$/g) | (6,36 mMol) | | (99,4%) |
| D4 | Jeffamin® D2000<br>(Texaco, USA<br>4,0 g (1 mVal $NH_2$/g) | 6,6 g B8<br>(2,0 mMol) | c | 10,2g<br>(96%) |
| D5 | KF-8003<br>(Shin Etsu, JP)<br>4,6 g (0,49 mVal $NH_2$/g) | 9,55 g B3<br>(2,25 mMol) | d | 13,9 g<br>(98%) |
| D6 | X-22-161B<br>(Shin Etsu, JP)<br>3,23 g (0,699 mVal $NH_2$/g)<br>$\overline{M}$~ 2900 | 4,0 g B5<br>(2,29 mMol) | e | 6,85 g<br>(95,4%) |

a = 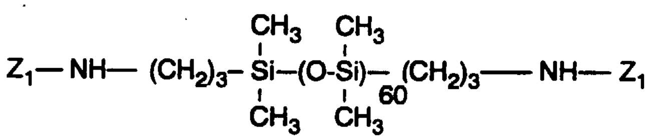

b = 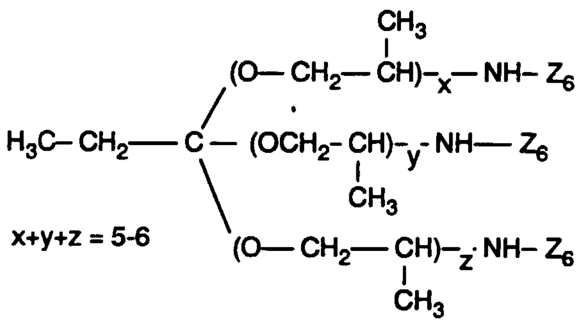

x+y+z = 5-6

c = 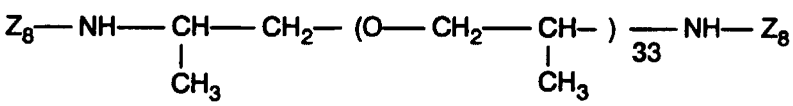

d = 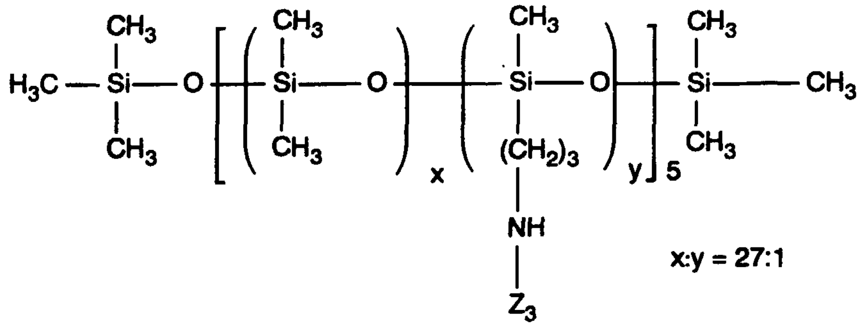

x:y = 27:1

e = 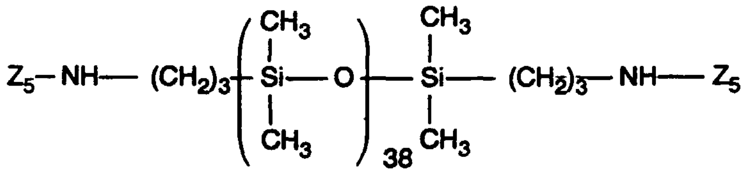

worin $Z_1$, $Z_3$, $Z_5$, $Z_6$ und $Z_8$ die entsprechenden Reste aus den Beispielen B1, B3, B5, B6 und B8 bedeuten.

Beispiel E1: (Herstellung eines polymerisierbaren Makromonomeren)

Unter Anwendung der in Beispiel D1 angeführten Reaktionsbedingungen werden 37,6g (0,01 Mol) des in Beispiel B2 beschriebenen OCN-funktionellen PVP-Phototelomeren mit 1,31 g 2-Hydroxyethylmethacrylat (HEMA) in 250 ml trockenem DMSO umgesetzt, wobei 10 mg Dibutylzinndilaurat als Katalysator zugesetzt werden. Nach dem Ausfällen in trockenem Diethylether und Trocknen bei $10^{-4}$ Torr werden 38,8 g eines weissen, pulvrigen Produkts erhalten.

## Patentansprüche

1. Vinyltelomere der Formel I,

$$O{=}C{=}N\text{-}PI^{*}\text{-}(A)_{p}R_{a} \qquad (I)$$

worin

PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
$R_a$ für eine einwertige Gruppe steht, die geeignet ist, als Kettenabbrecher einer Polymerisation zu dienen, und
p für eine ganze Zahl von 3 bis 500 steht.

2. Verfahren zur Herstellung einer Verbindung der Formel I gemäss Anspruch 1, dadurch gekennzeichnet, dass ein Photoinitiator der Formel B,

$$OCN\text{-}PI^{*}\text{-}R_{aa} \qquad (B)$$

worin PI* wie in Anspruch 1 definiert ist und $R_{aa}$ für den Teil eines Photoinititors steht, der bei einer Aufspaltung des Photoinitiators das weniger reaktive Radikal bildet, mit einem Vinylmonomer auf an sich bekannte Weise umgesetzt wird.

3. Verfahren gemäss Anspruch 2, dadurch gekennzeichnet, dass die Umsetzung unter Einwirkung von UV-Bestrahlung erfolgt.

4. Verfahren gemäss Anspruch 3, dadurch gekennzeichnet, dass eine Lösung enthaltend einen Photoinitiator der Formel B und eine zweite Lösung enthaltend ein Vinylmonomer wahrend der UV-Bestrahlung simultan zudosiert

werden.

**5.** Polymerisierbare Verbindungen der Formel C,

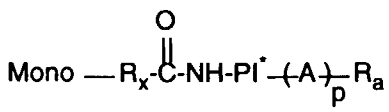

(C)

worin Mono für einen einwertigen Rest eines Vinylmonomeren steht, von dem die Gruppe $R_x$-H entfernt ist,

$R_x$ unabhängig voneinander für eine Bindung, -O-, -$NR_N$- oder -S- steht, worin $R_N$ für Wasserstoff oder $C_1$-$C_6$-Alkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
$R_a$ für eine einwertige Gruppe steht, die geeignet ist, als Kettenabbrecher einer Polymerisation zu dienen, und
p für eine ganze Zahl von 3 bis 500 steht.

**6.** Verfahren zur Herstellung einer Verbindung der Formel C gemäss Anspruch 5, dadurch gekennzeichnet, dass ein Vinyltelomer der Formel I, wie in Anspruch 1 definiert, auf an sich bekannte Weise mit einem Vinylmonomer umgesetzt wird, das mindestens eine Gruppe $R_x$-H enthält, worin $R_x$ für -O-,-$NR_N$- oder -S- steht, worin $R_N$ für Wasserstoff oder $C_1$-$C_6$-Alkyl steht.

**7.** Unvernetzte aber gegebenfalls vernetzbare Blockcopolymere der Formel D,

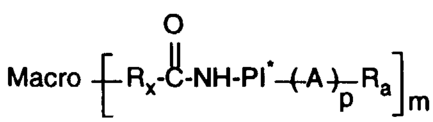

(D)

worin Macro für einen m-wertigen Rest eines Makromeren steht, von dem die Anzahl von m Gruppen $R_x$-H entfernt ist,

$R_x$ unabhängig voneinander für eine Bindung, -O-, -$NR_N$- oder -S- steht, worin $R_N$ für Wasserstoff oder $C_1$-$C_6$-Alkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
jedes $R_a$ unabhängig voneinander für eine einwertige Gruppe steht, die geeignet ist, als Kettenabbrecher einer Polymerisation zu dienen,
p unabhängig von m für eine ganze Zahl von 3 bis 500 steht und
m für eine ganze Zahl von 1 bis 100 steht.

**8.** Verfahren zur Herstellung einer Verbindung der Formel D gemäss Anspruch 7, dadurch gekennzeichnet, dass ein Makromer der Formel A:

$$\text{Macro-}(R_xH)_m \qquad (A)$$

worin Macro, $R_x$ und m wie in Anspruch 7 definiert sind, abgesehen davon, dass $R_x$ von einer Bindung verschieden ist, mit einem Vinyltelomeren der Formel I auf an sich bekannte Weise umgesetzt wird,

$$O{=}C{=}N\text{-}PI^{*}(A)_pR_a \qquad (I)$$

worin PI*, A, $R_a$ und p wie in Anspruch 7 definiert sind.

**9.** Polymere, bei denen es sich um die Polymerisationsprodukte eines polymerisationsfähigen Gemisches handelt, das die folgenden Bestandteile enthält:

a) eine polymerisationsfähige Verbindung der Formel C wie in Anspruch 5 definiert,

b) gegebenenfalls ein copolymerisierbares Vinylmonomer

c) einen copolymerisierbaren Vernetzer.

**10.** Polymere, bei denen es sich um die Polymerisationsprodukte eines polymerisationsfähigen Gemisches handelt, das die folgenden Bestandteile enthält:

aa) eine polymerisationsfähige Verbindung der Formel D wie in Anspruch 7 definiert, worin im Bestandteil -$(A)_p$- eine reaktive Gruppe enthalten ist,

bb) gegebenenfalls ein copolymerisierbares Vinylmonomer

cc) einen Vernetzer, der mit der reaktiven Gruppe im Bestandteil -$(A)_p$- der Verbindung der Formel D coreaktiv ist.

**11.** Verfahren zur Herstellung eines Polymeren gemäss Anspruch 9 oder 10, dadurch gekennzeichnet, dass die Komponenten a), b), und c), wie in Anspruch 9 definiert, oder die Komponenten aa), bb), und cc), wie in Anspruch 10 definiert, auf an sich bekannte Weise polymerisiert werden.

**12.** Formkörper enthaltend im wesentlichen ein Polymer gemäss Anspruch 9 oder 10.

**13.** Formkörper gemäss Anspruch 12, bei dem es sich um eine Kontaktlinse handelt.

**14.** Verwendung eines Vinyltelomeren der Formel I, wie in Anspruch 1 definiert, zur Beschichtung von Oberflächen.

**15.** Verwendung gemäss Anspruch 14, dadurch gekennzeichnet, dass es sich bei den Oberflächen um die Oberflächen von Kontatklinsen handelt.

## Claims

**1.** Vinyl telomers of formula I

$$O{=}C{=}N\text{-}PI^{*}-(A)_p-R_a \qquad (I)$$

wherein

PI* is a bivalent radical of a photoinitiator,
A is a substituted bivalent 1,2-ethylene radical derivable from a copolymerisable vinyl monomer by replacing the vinyl double bond with a single bond,
$R_a$ is a monovalent group that is suitable to act as a polymerisation chain-reaction terminator, and
p is an integer from 3 to 500.

**2.** A process for the preparation of a compound of formula I according to claim 1, which comprises reacting a photoinitiator of formula B

$$OCN\text{-}PI^{*}\text{-}R_{aa} \qquad (B)$$

wherein PI* is as defined in claim 1 and $R_{aa}$ is the moiety of a photoinitiator that forms the less reactive free radical on cleavage of the photoinitiator, with a vinyl monomer in a manner known *per se*.

3. A process according to claim 2, which comprises carrying out the reaction under the action of UV irradiation.

4. A process according to claim 3, wherein a solution comprising a photoinitiator of formula B and a second solution comprising a vinyl monomer are simultaneously metered in during UV irradiation.

5. A polymerisable compound of formula C

$$\text{Mono}-R_x\text{-}\overset{O}{\overset{\|}{C}}\text{-NH-PI}^*\!-\!(A)_p\!-R_a \qquad (C)$$

wherein Mono is a monovalent radical of a vinyl monomer from which the group $R_x$-H has been removed,

each $R_x$, independently of the others, is a bond, -O-, -$NR_N$- or -S- wherein $R_N$ is hydrogen or $C_1$-$C_6$-alkyl,
PI* is a bivalent radical of a photoinitiator,
A is a substituted bivalent 1,2-ethylene radical derivable from a copolymerisable vinyl monomer by replacing the vinyl double bond with a single bond,
$R_a$ is a monovalent group that is suitable to act as a polymerisation chain-reaction terminator, and
p is an integer from 3 to 500.

6. A process for the preparation of a compound of formula C according to claim 5, wherein a vinyl telomer of formula I as defined in claim 1 is reacted in a manner known *per se* with a vinyl monomer that contains at least one $R_x$-H group, wherein $R_x$ is —O-, -$NR_N$- or -S-, $R_N$ being hydrogen or lower alkyl.

7. A block copolymer of formula D that is uncrosslinked, but if desired, crosslinkable,

$$\text{Macro}-\left[R_x\text{-}\overset{O}{\overset{\|}{C}}\text{-NH-PI}^*\!-\!(A)_p\!-R_a\right]_m \qquad (D)$$

wherein Macro is an m-valent radical of a macromer from which the number m of groups $R_x$-H has been removed,

each $R_x$, independently of the others, is a bond, -O-, -$NR_N$- or -S- wherein $R_N$ is hydrogen or $C_1$-$C_6$-alkyl,
PI* is a bivalent radical of a photoinitiator,
A is a substituted bivalent 1,2-ethylene radical derivable from a copolymerisable vinyl monomer by replacing the vinyl double bond with a single bond,
each $R_a$, independently of the others, is a monovalent group that is suitable to act as a polymerisation chain-reaction terminator, and
p, independently of m, is an integer from 3 to 500 and
m is an integer from 1 to 100.

8. A process for the preparation of a compound of formula D according to claim 7, which comprises reacting a macromer of formula A:

$$\text{Macro-}(R_xH)_m \qquad (A),$$

wherein Macro, $R_x$ and m are as defined in claim 7 except that $R_x$ is other than a bond, in a manner known *per se* with a vinyl telomer of formula I

$$O{=}C{=}N\text{-}PI^{*}\!-\!(A)_p\!-\!R_a \qquad (I)$$

wherein PI*, A, $R_a$, and p are as defined in claim 7.

**9.** A polymer, which is a polymerisation product of a polymerisable mixture that comprises the following components:

a) a polymerisable compound of formula C as defined in claim 5,
b) if desired a copolymerisable vinyl monomer,
c) a copolymerisable crosslinker;

**10.** A polymer, which is a polymerisation product of a polymerisable mixture that comprises the following components:

aa) a polymerisable compound of formula D as defined in claim 7, in which a reactive group is present in the component $-(A)_p-$,
bb) if desired a copolymerisable vinyl monomer,
cc) a crosslinker that is co-reactive with the reactive group in the component $-(A)_p-$ of the compound of formula D.

**11.** A process for the preparation of a polymer according to claim 9 or 10, which comprises polymerising components a), b) and c) as defined in claim 9, or components aa), bb) and cc) as defined in claim 10, in a manner known *per se*.

**12.** A moulding comprising essentially a polymer according to claim 9 or 10.

**13.** A moulding according to claim 12, which is a contact lens.

**14.** The use of a vinyl telomer of formula I as defined in claim 1 for the coating of surfaces.

**15.** Use according to claim 14, wherein the surfaces are the surfaces of contact lenses.

## Revendications

**1.** Des télomères vinyliques de formule I

$$O{=}C{=}N\text{-}PI^{*}(A)_pR_a \qquad (I)$$

où

PI* signifie un reste bivalent d'un photoinitiateur,
A signifie un reste 1,2-éthylène bivalent substitué pouvant être dérivé d'un monomère vinylique copolymérisable pour remplacer la double liaison vinylique par une simple liaison,
$R_a$ signifie un groupe monovalent qui est approprié pour servir de rupteur de la chaîne d'une polymérisation, et
p signifie un nombre entier de 3 à 500.

**2.** Un procédé de préparation d'un composé de formule I selon la revendication 1, caractérisé en ce qu'on fait réagir de manière connue un photo-initiateur de formule B

$$OCN\text{-}PI^{*}\text{-}R_{aa} \qquad (B)$$

où PI* est tel que défini à la revendication 1 et $R_{aa}$ signifie une partie d'un photoinitiateur qui forme le groupe le moins réactif lors de la scission du photoinitiateur, avec un monomère vinylique.

**3.** Un procédé selon la revendication 2, caractérisé en ce qu'on effectue la réaction sous l'action du rayonnement UV.

**4.** Un procédé selon la revendication 3, caractérisé en ce que, pendant le rayonnement UV, on ajoute simultanément par dosage, une solution contenant un photoinitiateur de formule B et une seconde solution contenant un monomère vinylique.

**5.** Les composés polymérisables de formule C

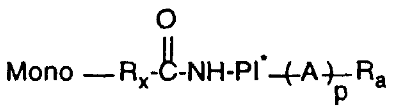

(C)

où Mono signifie un reste monovalent d'un monomère vinylique duquel le groupe $R_x$-H a été retiré,

$R_x$ signifie indépendamment de l'autre, une liaison, -O-, -$NR_N$- où -S- où $R_N$ signifie l'hydrogène ou un groupe $C_1$-$C_6$-alkyle,
PI* signifie un reste bivalent d'un photoinitiateur,
A signifie un reste 1,2-éthylène bivalent substitué pouvant être dérivé d'un monomère vinylique copolymérisable pour remplacer la double liaison vinylique par une simple liaison,
$R_a$ signifie un groupe monovalent qui est approprié pour servir de rupteur de la chaîne d'une polymérisation, et
p signifie un nombre entier de 3 à 500.

**6.** Un procédé de préparation d'un composé de formule C selon la revendication 5, caractérisé en ce qu'on fait réagir de manière connue un télomère vinylique de formule I tel que défini à la revendication 1, avec un monomère vinylique qui contient au moins un groupe $R_x$-H où $R_x$ signifie -O-, -$NR_N$- ou -S-, où $R_N$ signifie l'hydrogène ou un groupe $C_1$-$C_6$-alkyle.

**7.** Des copolymères séquencés de formule D non réticulés, mais éventuellement réticulables

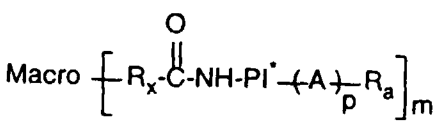

(D)

où Macro signifie un reste m-valent d'un macromère duquel le nombre m de groupes $R_x$-H a été retiré,

$R_x$ signifie indépendamment de l'autre, une liaison, -O-, -$NR_N$- ou -S- où $R_N$ signifie l'hydrogène ou un groupe $C_1$-$C_6$-alkyle,
PI* signifie un reste bivalent d'un photoinitiateur,
A signifie un reste 1,2-éthylène bivalent substitué pouvant être dérivé d'un monomère vinylique copolymérisable pour remplacer la double liaison vinylique par une simple liaison,
chaque $R_a$ signifie indépendamment de l'autre, un groupe monovalent qui est approprié pour servir de rupteur de la chaîne d'une polymérisation,
p signifie indépendamment de m un nombre entier de 3 à 500, et
m signifie un nombre entier de 1 à 100.

**8.** Un procédé de préparation d'un composé de formule D selon la revendication 7, caractérisé en ce qu'on fait réagir de manière connue un macromère de formule A:

$$Macro\text{-}(R_xH)_m \qquad (A)$$

où Macro, $R_x$ et m sont tels que définis à la revendication 7, sauf que $R_x$ a une signification autre qu'une liaison, avec un télomère vinylique de formule I

$$O{=}C{=}N\text{-}PI^*(A)_pR_a \qquad (I)$$

où PI*, A, $R_a$ et p sont tels que définis à la revendication 7.

**9.** Des polymères qui sont des produits de polymérisation d'un mélange polymérisable comprenant les composants suivants:

a) un composé polymérisable de formule C tel que défini à la revendication 5,
b) éventuellement, un monomère vinylique copolymérisable,
c) un agent de réticulation copolymérisable.

**10.** Des polymères qui sont des produits de polymérisation d'un mélange polymérisable comprenant les composants suivants:

aa) un composé polymérisable de formule D tel que défini à la revendication 7, où dans le composant $-(A)_p-$ un groupe réactif est contenu,
bb) éventuellement, un monomère vinylique copolymérisable,
cc) un agent de réticulation qui est co-réactif avec le groupe réactif dans le composant $-(A)_p-$ du composé de formule D.

**11.** Un procédé de préparation d'un polymère selon la revendication 9 ou la revendication 10, caractérisé en ce qu'on polymérise de manière connue les composants a), b) et c) tels que définis à la revendication 9, ou les composants aa), bb) et cc) tels que définis à la revendication 10.

**12.** Un moulage contenant essentiellement un polymère selon la revendication 9 ou la revendication 10.

**13.** Un moulage selon la revendication 12, qui est une lentille de contact.

**14.** L'utilisation d'un télomère vinylique de formule I tel que défini à la revendication 1, pour l'enrobage de surfaces.

**15.** L'utilisation selon la revendication 14, caractérisée en ce que les surfaces sont des surfaces de lentilles de contact.